# EUROPÄISCHE PATENTSCHRIFT

(11) **EP 3 843 822 B1**
(45) Veröffentlichungstag und Bekanntmachung des Hinweises auf die Patenterteilung: **10.04.2024**
(21) Anmeldenummer: 19759359.3
(22) Anmeldetag: 26.08.2019
(51) Int. Cl.: A61M 16/00, A61M 16/08, A61M 16/10, A61M 16/16

(54) **BEATMUNGSVORRICHTUNG MIT VERBESSERTER BEFEUCHTUNG DES ATEMGASES**
RESPIRATORY DEVICE WITH IMPROVED HUMIDIFICATION OF THE RESPIRATION GAS
DISPOSITIF RESPIRATOIRE AVEC HUMIDIFICATION AMÉLIORÉE DU GAZ RESPIRATOIRE

(30) Priorität: 28.08.2018 DE 102018214556
(43) Veröffentlichungstag der Anmeldung: 07.07.2021
(73) Patentinhaber: Hamilton Medical AG, 7402 Bonaduz (CH)
(72) Erfinder: DINO, De-stefani, 7000 Chur (CH)
(74) Vertreter: Ruttensperger Lachnit Trossin Gomoll
(86) Internationale Anmeldenummer: PCT/EP2019/072731
(87) Internationale Veröffentlichungsnummer: WO 2020/043672

(56) Entgegenhaltungen:
- EP-A1- 2 143 459
- DE-A1-102017 217 859
- US-A1- 2013 073 013

## Beschreibung

Die vorliegende Erfindung betrifft eine Beatmungsvorrichtung zur künstlichen Beatmung eines Patienten, umfassend:
- eine Atemgas-Quellenanordnung, welche ein inspiratorisches Atemgas zur künstlichen Beatmung des Patienten bereitstellt,
- eine Strömungsveränderungsvorrichtung, welche dazu ausgebildet ist, einen inspiratorischen Atemgasfluss zu erzeugen und betragsmäßig zu verändern,
- eine Befeuchtungsvorrichtung, welche dazu ausgebildet ist, die absolute Feuchtigkeit des inspiratorischen Atemgasflusses betragsmäßig zu erhöhen, wobei die Befeuchtungsvorrichtung hierzu einen Flüssigkeitsvorrat und eine leistungsveränderliche Verdampfungsvorrichtung aufweist,
- eine Atemgasleitungsanordnung mit einem im Betrieb dem Patienten näher gelegenen proximalen Längsende und mit einem im Betrieb vom Patienten weiter entfernt gelegenen distalen Längsende, um den inspiratorischen Atemgasfluss von der Befeuchtungsvorrichtung zum Patienten hin zu fördern,
- einen proximalen Temperatursensor, welcher dazu ausgebildet ist, die Temperatur des Atemgasflusses im proximalen Längsendbereich der Atemgasleitungsanordnung zu erfassen,
- eine Feuchte-Sensoranordnung, welche dazu ausgebildet ist, wenigstens einen Feuchte-Zustandswert des inspiratorischen Atemgasflusses zu erfassen, der mittelbar oder unmittelbar eine absolute Feuchtigkeit des inspiratorischen Atemgasflusses repräsentiert,
- einen Flusssensor, welcher dazu ausgebildet ist, den Atemgasfluss betragsmäßig zu erfassen,
- eine Steuervorrichtung, welche signalübertragungsmäßig mit dem proximalen Temperatursensor, der Feuchte-Sensoranordnung und dem Flusssensor verbunden ist und welche dazu ausgebildet ist, die Betriebsleistung der Verdampfungsvorrichtung abhängig von einer vorgegebenen Soll-Feuchtigkeit des Atemgases und abhängig von Signalen des proximalen Temperatursensors, der Feuchte-Sensoranordnung und des Flusssensors zu steuern.

Eine ähnliche Beatmungsvorrichtung ist aus der US 2013/0073013 A1 bekannt. Die bekannte Beatmungsvorrichtung nutzt Umgebungsluft als Atemgas-Quelle sowie eine Kombination aus Gebläse und Ventil, um die Strömung eines Atemgasflusses in der Beatmungsvorrichtung betragsmäßig zu verändern. Eine Verdampfer-Befeuchtungsvorrichtung ist vorgesehen, um das Atemgas erforderlichenfalls zu befeuchten. Ein in der Verdampfer-Befeuchtungsvorrichtung vorhandener Wasservorrat wird vom Atemgasfluss überströmt und nimmt dabei über dem Wasserspiegel des Wasservorrats vorhandenen Wasserdampf mit.

DE102017217859 beschreibt eine Beatmungsvorrichtung mit einer Konditionierungseinrichtung. Die aus der US 2013/0073013 A1 bekannte Beatmungsvorrichtung umfasst weiter eine beheizbare Atemgasleitungsanordnung, um durch Wärmezufuhr an die Atemgasleitungsanordnung ein unerwünschtes Auskondensieren von Feuchtigkeit im Atemgas zu verhindern.

Stromabwärts der Befeuchtungsvorrichtung der bekannten Beatmungsvorrichtung, aber noch vor Beginn der Heizstrecke der Atemgasleitungsanordnung, also im Bereich des distalen Endes der Atemgasleitungsanordnung, sind ein Flusssensor zur Ermittlung des Atemgasflusses, ein Temperatursensor zur Erfassung der Temperatur des Atemgases und ein Feuchtigkeitssensor zu Erfassung der Feuchtigkeit des Atemgases vorgesehen. Außerdem sind Sensoren zur Erfassung der Umgebungstemperatur und der Umgebungsluftfeuchtigkeit vorgesehen.

Über eine Eingabe/Ausgabevorrichtung können Betriebsparameter der Beatmungsvorrichtung durch einen Arzt oder durch Pflegepersonal eingestellt werden.

Eine Steuervorrichtung der bekannten Beatmungsvorrichtung steuert die Beatmungsvorrichtung in nicht näher bezeichneter Weise. Die Steuervorrichtung ermittelt ausgehend von der erfassten Umgebungstemperatur und der erfassten Umgebungsluftfeuchtigkeit, der erfassten Temperatur des Atemgasflusses und der erfassten Feuchtigkeit des Atemgasflusses sowie weiter abhängig von Betriebsdaten und konstruktiven Daten der Atemgasleitungsanordnung eine Soll-Temperatur für den Betrieb der Heizung der Atemgasleitungsanordnung. Die Soll-Temperatur wird dabei derart gewählt, dass die Temperatur in der Atemgasleitungsanordnung über der jeweiligen Taupunkttemperatur liegt. Die Steuervorrichtung steuert die Heizung der Atemgasleitungsanordnung in Abhängigkeit von der gewählten Soll-Temperatur. Die Taupunkttemperatur wird gemäß der Offenbarung der US 2013/0073013 A1 aus den verfügbaren psychrometrischen Daten gewonnen, also aus der erfassten Temperatur und der erfassten relativen Feuchtigkeit des Atemgasflusses.

Eine andere Beatmungsvorrichtung ist aus der EP 2 143 459 B1 bekannt. Auch diese offenbart eine Atemgasleitungsanordnung mit einer Atemgasquelle und mit einem Gebläse als Strömungsveränderungsvorrichtung. Die aus der EP 2 143 459 B1 bekannte Beatmungsvorrichtung weist ebenfalls eine Verdampfer-Befeuchtungsvorrichtung auf. Auch diese hat einen Wasservorrat, welcher im Betrieb vom Atemgasfluss überströmt wird, wobei der Atemgasfluss während des Überströmens befeuchtet wird.

Der von der Befeuchtungsvorrichtung bis zum proximalen Längsende der Atemgasleitungsanordnung verlaufende Abschnitt der Atemgasleitungsanordnung der bekannten Beatmungsvorrichtung ist beheizbar. In diesem Abschnitt sind in dem der Befeuchtungsvorrichtung näher gelegenen Längsendbereich der Atemgasleitungsanordnung ein Flusssensor und ein Temperatursensor zur Ermittlung der Temperatur und des betragsmäßigen Flusses von Atemgas angeordnet. Ebenso ist am proximalen Längsende der Atemgasleitungsanordnung, an welches eine Patientenschnittstelle zur Verbindung der Atemgasleitungsanordnung mit einem zu beatmenden Patienten ankoppelbar ist, ein proximaler Temperatursensor angeordnet.

An einer Steuervorrichtung der bekannten Beatmungsvorrichtung kann eine Bedienperson eine gewünschte Atemgastemperatur oder ein gewünschtes Feuchtigkeitsniveau des von der Beatmungsvorrichtung gelieferten Atemgases einstellen. Die Steuervorrichtung ist datenübertragungsmäßig mit den beiden Temperatursensoren und mit dem Flusssensor verbunden und erhält so deren Erfassungswerte.

Unter Verwendung von in einem Datenspeicher hinterlegten Tabellen, Formeln oder Kennfeldern steuert die Steuervorrichtung nach Maßgabe einer erforderlichen Atemgasfeuchtigkeit sowie eines sensorisch ermittelten Atemgasflusses und der sensorisch ermittelten Atemgastemperatur die Energiezufuhr zu einer Heizplatte der Befeuchtungsvorrichtung, umso einen Atemgasfluss mit einer gewünschten Atemgasfeuchtigkeit und einer gewünschten Atemgastemperatur bereitzustellen. Die erforderliche Atemgasfeuchtigkeit ist entweder die durch die Bedienperson eingestellte Feuchtigkeit oder eine in einem Datenspeicher vorab hinterlegte Feuchtigkeit.

Wie allgemein bekannt ist, ist bei künstlicher Beatmung die Zuführung eines Atemgasflusses mit korrektem Feuchtegrad zum Patienten von besonderer Wichtigkeit. Ist das zugeführte Atemgas zu trocken, dehydriert der Patient im Laufe der künstlichen Beatmung. Ist das zugeführte Atemgas zu feucht, droht Wasser aus dem Atemgas auszukondensieren und in flüssiger Form in die Lunge des Patienten zu gelangen, wo es den gewünschten metabolischen Gasaustausch behindert.

Selbst wenn das aus dem Atemgas auskondensierte Wasser nicht bis in die Lunge des Patienten gelangt, kann es an Wänden der Atemgasleitungsanordnung oder/und von Sensoren, wie beispielsweise einem proximalen Flusssensor, niederschlagen und so die Messgenauigkeit der Sensoren herabsetzen.

Entscheidend ist dabei eher die relative Feuchte des Atemgases als dessen absolute Feuchte. Die absolute Feuchte gibt an, welche Menge an Wasser in einem Referenzvolumen an Luft gelöst ist. Die relative Feuchte ist ein Maß dafür, wie viel Feuchtigkeit ein vorbestimmtes Referenzvolumen an Luft noch aufnehmen und beispielsweise dem Patienten entziehen kann oder wie nahe das Atemgas an seiner Feuchtigkeits-Sättigung ist. Bei nahezu mit Feuchtigkeit gesättigtem Atemgas können bereits geringe Änderungen der Atemgastemperatur zu einem höchst unerwünschten Auskondensieren (in der Fachwelt auch als "rain out" bezeichnet) von Flüssigkeit aus dem Atemgas führen.

Aus der DE 10 2017 217 859 A1 ist eine Beatmungsvorrichtung bekannt, welche in der Lage ist, aus fluss- und drucksignalbasierter Erfassung auf einen Fehler eines Durchflusssensors der Beatmungsvorrichtung zu schließen. Die bekannte Beat mungsvorrichtung ist außerdem in der Lage, ein inspiratorisches Atemgas zu befeuchten und zu temperieren.

Es ist daher Aufgabe der vorliegenden Erfindung, eine Beatmungsvorrichtung der eingangs genannten Art derart weiterzubilden, dass sie einen Patienten möglichst exakt und möglichst stabil über einen langen Beatmungszeitraum hinweg mit korrekt befeuchtetem Atemgas beatmen kann.

Diese Aufgabe wird erfindungsgemäß gelöst durch eine Beatmungsvorrichtung, wie sie eingangs der Anmeldung beschrieben ist, bei welcher zusätzlich die Feuchte-Sensoranordnung in Inspirationsrichtung des Atemgasflusses stromaufwärts der Befeuchtungsvorrichtung angeordnet ist und dazu ausgebildet ist, den wenigstens einen Feuchte-Zustandswert stromaufwärts der Befeuchtungsvorrichtung zu erfassen, wobei die Beatmungsvorrichtung wenigstens einen Umgebungstemperatursensor aufweist, welcher dazu ausgebildet ist, die Temperatur der Umgebung der Beatmungsvorrichtung zu erfassen, wobei die Steuervorrichtung signalübertragungsmäßig mit dem wenigstens einen Umgebungstemperatursensor verbunden ist und dazu ausgebildet ist, die Betriebsleistung der Verdampfungsvorrichtung zusätzlich zu den bereits genannten Abhängigkeiten auch abhängig von Signalen des wenigstens einen Umgebungstemperatursensors zu steuern.

Durch die Anordnung der Feuchte-Sensoranordnung in Inspirationsrichtung stromaufwärts der Befeuchtungsvorrichtung kann die Feuchte-Sensoranordnung die Feuchte des Atemgases im Ausgangszustand, also vor jeglicher Befeuchtungsmaßnahme, erfassen. Somit kann die Steuervorrichtung besonders genau den Befeuchtungsbedarf des Atemgases ermitteln.

Dies ist überraschenderweise vorteilhafter als die aus der US 2013/0073013 A1 bekannte Anordnung der Feuchte-Sensoranordnung stromabwärts der Befeuchtungsvorrichtung, von welcher man erwarten würde, dass sie eine Feedback-Regelung der Atemgasfeuchtigkeit auf Grundlage des Erfassungswerts der Feuchte-Sensoranordnung ermöglicht. Tatsächlich erfolgt jedoch eine Änderung der Atemgasfeuchtigkeit durch Änderung der Energiezufuhr an eine Heizplatte der Befeuchtungsvorrichtung sehr langsam und träge, sodass ein Regelkreis mit kleiner werdenden Abständen zwischen Soll-Feuchte und Ist-Feuchte immer schwieriger mit ausreichender Genauigkeit auszuführen ist.

Es hat sich daher herausgestellt, dass es zur Regelung der Atemgasfeuchtigkeit vorteilhafter ist, die Atemgasfeuchtigkeit zu erfassen, bevor das Atemgas die Befeuchtungsvorrichtung erreicht, umso den tatsächlichen Befeuchtungsbedarf zu ermitteln und die Befeuchtungsvorrichtung gemäß diesem Bedarf zu betreiben. Dies führt zu einer stärkeren und schnelleren Annäherung der tatsächlichen Ist-Atemgasfeuchtigkeit an die gewünschte Soll-Feuchtigkeit als eine etwaige Regelung auf Grundlage einer Erfassung der Atemgasfeuchtigkeit nach der Beatmungsvorrichtung.

Durch die Erfassung der Umgebungstemperatur mit dem Umgebungstemperatursensor steht der Steuervorrichtung eine Größe zur Verfügung, welche ein Maß für thermische Verluste der Befeuchtungsvorrichtung oder/und des Atemgases an die Umgebung ist. Dadurch ist es der Steuervorrichtung möglich, die Betriebsleistung der Verdampfungsvorrichtung nicht nur anhand der unmittelbaren Betriebsparameter, wie Atemgasfluss, Temperatur des Atemgases am proximalen Temperatursensor und Atemgasfeuchtigkeit zu ermitteln, sondern zur Ermittlung dieser Betriebsleistung etwaige Verlustleistungen an die Umgebung zu berücksichtigen.

Die Steuervorrichtung ist daher dazu ausgebildet, die vom Umgebungstemperatursensor erfasste Umgebungstemperatur als Korrekturgröße für eine Korrektur der ermittelten Betriebsleistung der Verdampfungsvorrichtung bezüglich etwaiger thermischer Umgebungsverluste an die Außenumgebung zu verwenden.

Die Korrektur der der Verdampfungsvorrichtung von der Steuervorrichtung zugeführten Betriebsleistung um thermische Umgebungsverluste ist gerade für die Notfallmedizin und Erstversorgung von besonderer Wichtigkeit. Beatmungsvorrichtungen, wie die vorliegend diskutierte werden nicht nur in parametrisch vorhersehbaren klinischen Umgebungen eingesetzt, sondern auch in Notfall-Transportfahrzeugen, bis hin zu Transporthelikoptern, deren Umgebungsbedingungen alles andere als konstant und vorhersehbar sind. Man denke hier an einen Extremfall, wie beispielsweise einen zur Bergrettung in einem Transporthelikopter eingesetzten Notarzt, der im Winter in alpiner Umgebung schwerverletzte Unfallopfer in Höhen von 3000 bis 4800 m ü. d. M. bergen und sofort lebenserhaltend versorgen können soll.

Die Steuervorrichtung kann die Betriebsleistung der Verdampfungsvorrichtung ausgehend von den genannten Parametern anhand von empirisch ermittelten Zusammenhängen steuern, die vorab für eine Vielzahl von Parameterkombinationen im Labor ermittelt wurden. Die Zusammenhänge können in einem von der Steuervorrichtung abfragbaren Datenspeicher als Formelzusammenhang, Kennfeld oder/und Tabelle hinterlegt sein.

Dabei kann die Einstellung der Atemgasfeuchtigkeit durch die Steuervorrichtung in mehreren Schritten erfolgen. Zunächst kann die Steuervorrichtung ausgehend von einer gewünschten Atemgastemperatur am Messort des proximalen Temperatursensors (nachfolgend auch als "proximale Atemgastemperatur" bezeichnet) sowie weiter ausgehend von einem mengenmäßig gewünschten Atemgasfluss eine Soll-Atemgasfeuchtigkeit ermitteln. Diese Ermittlung kann wiederum auf Grundlage von empirisch bestimmten und in einem von der Steuervorrichtung abfragbaren Datenspeicher hinterlegten Datenzusammenhängen erfolgen. Ausgehend von der so bestimmten Soll-Atemgasfeuchtigkeit als der Zielgröße kann dann aufgrund wiederum von empirisch bestimmten und in einem Datenspeicher hinterlegten Zusammenhängen anhand von der stromaufwärts der Beatmungsvorrichtung erfassten Ist-Atemgasfeuchtigkeit und weiteren der genannten Parameter die Betriebsleistung der Verdampfungsvorrichtung ermittelt werden.

Die Umgebungstemperatur als Verlust-Korrekturgröße kann unmittelbar bei der Ermittlung der Betriebsleistung berücksichtigt werden oder es kann eine Betriebsleistung anhand der eingangs genannten Parameter ermittelt und diese nach Maßgabe der erfassten Umgebungstemperatur anschließend korrigiert werden.

Zwar ist, wie oben ausgesagt ist, die relative Feuchte des Atemgases für die physiologische Verträglichkeit der Beatmung entscheidender als die absolute Feuchte. Für die Ermittlung der Betriebsleistung der Verdampfungsvorrichtung ist jedoch die absolute Feuchte des Atemgases geeigneter, da dann die in einem vorbestimmten Atemgasvolumen enthaltene Wassermasse als solche bekannt ist. Hieraus lässt sich sehr einfach ermitteln, welche Differenz-Wassermasse dem Atemgas zugeführt werden muss, damit das Atemgas korrekt befeuchtet ist. Die zur Verdampfung einer bekannten Masse an Wasser in einer vorbestimmten Zeit ermittelbare notwendige Leistung ist dann die noch hinsichtlich Umgebungsverlusten unkorrigierte Betriebsleistung. Die relative Feuchte des Atemgases für sich alleine genommen bedeutete dagegen nur das Verhältnis der aktuellen Atemgasfeuchtigkeit zur gesättigten Atemgasfeuchtigkeit, ohne dass hieraus die fehlende Differenz-Wassermenge ermittelbar wäre.

Der absoluten Feuchtigkeit des Atemgases steht jedoch dessen relative Feuchtigkeit bei gleichzeitiger Kenntnis der Atemgastemperatur einerseits und der Taupunktkurve der zu verdampfen Flüssigkeit, in der Regel Wasser, andererseits gleich. Daher kann die Feuchte-Sensoranordnung entweder einen Sensor zu Ermittlung der absoluten Atemgasfeuchtigkeit aufweisen oder einen Sensor zur Ermittlung der relativen Atemgasfeuchtigkeit und einen Temperatursensor aufweisen, sofern in einem von der Steuervorrichtung abfragbaren Datenspeicher die Taupunktkurve der zu verdampfen Flüssigkeit oder ein der Taupunktkurve entsprechender Datenzusammenhang hinterlegt ist.

Der sensorischen Ermittlung eines angegebenen Parameters im Sinne der vorliegenden Ermittlung steht die unmittelbare Ermittlung einer anderen Größe als des angegebenen Parameters gleich, wenn diese andere Größe den angegebenen Parameter auf Grundlage eines bekannten Zusammenhangs, etwa eines Kalibrationszusammenhangs, repräsentiert.

In der vorliegenden Anmeldung sind die Begriffe "Feuchtigkeit" und "Feuchte" synonym gebraucht.

Die empirische Ermittlung von Datenzusammenhängen stellt zwar einen Aufwand dar, da in einem Labor die genannten Einflussparameter kontrolliert im Rahmen ihres erwarteten Betriebswertebereichs variiert und hinsichtlich ihres Einflusses auf die Zielparameter erfasst werden müssen. Jedoch muss dieser Aufwand für baugleiche Beatmungsvorrichtungen nur einmalig erbracht werden. Die so für eine konstruktive Ausgestaltung von Beatmungsvorrichtungen ermittelten Datenzusammenhänge können dann für eine Vielzahl baugleicher Beatmungsvorrichtungen verwendet werden. Es reicht dann aus, stichprobenartig zu überprüfen, ob ein bereits ermittelter Datenzusammenhang für eine konkrete Beatmungsvorrichtung zutrifft oder nicht.

Der Flusssensor kann den Betrag wenigstens des inspiratorischen Atemgasflusses, ebenso wie der proximale Temperatursensor die Temperatur, im proximalen Längsendbereich der Atemgasleitungsanordnung erfassen, sodass die Befeuchtung des Atemgases anhand von Parametern erfolgt, die möglichst nahe beim Patienten liegen.

Zur Definition einer gewünschten Beatmungssituation kann die Beatmungsvorrichtung eine Eingabevorrichtung zur Eingabe wenigstens einer Soll-Temperatur des Atemgasflusses im proximalen Längsendbereich der Atemgasleitungsanordnung oder/und zur Eingabe wenigstens einer Soll-Feuchtigkeit des Atemgases oder/und zur Eingabe wenigstens eines Soll-Atemgasflusses aufweisen. Es können auch nur einige der genannten Parameter an der Eingabevorrichtung eingebbar sein, während andere der genannten Parameter anhand von empirisch ermittelten Datenzusammenhängen nach Maßgabe der eingegebenen oder/und weiterer Parameter ermittelbar sind.

Beispielsweise kann die Beatmungsvorrichtung zur Vereinfachung ihrer Bedienung eine Eingabevorrichtung zur Eingabe einer Soll-Temperatur des Atemgasflusses im proximalen Längsendbereich der Atemgasleitungsanordnung aufweisen und kann weiter einen von der Steuervorrichtung abfragbaren Datenspeicher aufweisen, in welchem ein, vorzugsweise empirisch ermittelter, Zusammenhang zwischen Temperaturwerten des Atemgasflusses im proximalen Längsendbereich der Atemgasleitungsanordnung und zugeordneten Soll-Feuchtigkeitswerten des Atemgases gespeichert ist. Dann reicht es aus, nur die proximale Atemgasfluss-Temperatur einzugeben, damit die Steuervorrichtung die zugeordnete Soll-Feuchtigkeit des Atemgases bestimmen kann.

Zur Vermeidung von unerwünschtem Niederschlag von Flüssigkeit an den Wänden der Atemgasleitungsanordnung kann wenigstens ein inspiratorischer Leitungsabschnitt der Atemgasleitungsanordnung eine Leitungsheizvorrichtung aufweisen. Mit dieser Leitungsheizvorrichtung kann der inspiratorische Leitungsabschnitt beheizt werden. Damit kann verhindert werden, dass die Temperatur einer Wand des inspiratorischen Leitungsabschnitts unter die Taupunkttemperatur des im Leitungsabschnitt strömenden Atemgases sinkt. Die Steuervorrichtung kann dann dazu ausgebildet sein, die Betriebsleistung der Leitungsheizvorrichtung abhängig von einer vorgegebenen Soll-Temperatur des Atemgasflusses im proximalen Längsendbereich der Atemgasleitungsanordnung zu steuern. Bei der Ermittlung der an die Leitungsheizvorrichtung zuzuführenden Energie bzw. Leistung kann die Steuervorrichtung außerdem die erfasste Umgebungstemperatur berücksichtigen. Dies vereinfacht den Regelkreis zur Beheizung des Leitungsabschnitts erheblich, da in der Regel die Atemgasleitungsanordnung an ihrer Außenseite von Umgebungsluft mit Umgebungstemperatur benetzt ist. Wird die Umgebungstemperatur bei der Beheizung des Leitungsabschnitts berücksichtigt, können unerwünschte Temperaturschwankungen an der beheizten Wand des inspiratorischen Leitungsabschnitts vermieden werden. Die Temperatur des beheizten Leitungsabschnitts kann so mit besserer Konstanz gehalten werden.

Die Atemgas-Quellenanordnung kann als eine Atemgasquelle eine Ansaugöffnung aufweisen, durch welche hindurch Umgebungsluft oder Gas aus einem vorbestimmten Gasvorrat angesaugt werden kann. Die Atemgas-Quellenanordnung kann zusätzlich oder alternativ einen Gasvorrat als Atemgasquelle aufweisen, beispielsweise als Vorratsbehälter oder als Anschlussformation zum Anschluss einer Versorgungsleitung, welche die Beatmungsvorrichtung mit einem lokal installierten Gasvorrat verbindet, wie dies häufig in Kliniken der Fall ist. Um die Möglichkeit bereitzustellen, unterschiedliche Gase zu einem Atemgas zu mischen, kann die Atemgas-Quellenanordnung eine Mehrzahl von einzelnen Atemgasquellen, wie die oben genannten, aufweisen. Dabei können die unterschiedlichen zu mischenden Gase aufgrund von Bereitstellung und Entspannung unterschiedliche Temperaturen oder/und unterschiedliche Feuchtigkeit aufweisen. Um sicher zu gehen, dass die Feuchte-Sensoranordnung die Feuchtigkeit des tatsächlich dem Patienten zugeführten Atemgases erfasst, ist die Feuchte-Sensoranordnung bevorzugt in Inspirationsrichtung des Atemgasflusses stromabwärts der Atemgas-Quellenanordnung angeordnet. Aus den genannten Gründen wird besonders bevorzugt in Inspirationsrichtung stromabwärts der Befeuchtungsvorrichtung kein Atemgasbestandteil mehr dem aus der Befeuchtungsvorrichtung austretenden Atemgasfluss zugefügt.

Zur Erfassung und zur Steuerung der der Verdampfungsvorrichtung zugeführten Energie bzw. Leistung kann die Steuervorrichtung mit der Verdampfungsvorrichtung signalübertragungsmäßig verbunden sein. Zusätzlich oder alternativ kann die Steuervorrichtung signalübertragungsmäßig mit einer Energieversorgung der Verdampfungsvorrichtung verbunden sein. Beispielsweise kann die Steuervorrichtung unmittelbar dazu ausgebildet sein, einen der Verdampfungsvorrichtung zugeführten elektrischen Strom und die dabei anliegende elektrische Spannung erfassen. Sofern die unmittelbare Ausbildung der Steuervorrichtung zur Erfassung von Energie, insbesondere elektrische Energie, einen zu großen Aufwand bedeutet, kann die Steuervorrichtung signalübertragungsmäßig mit wenigstens einem Energiesensor verbunden sein, welcher dazu ausgebildet ist, eine der Verdampfungsvorrichtung zugeführte Energie zu erfassen. Die der Verdampfungsvorrichtung aktuell zugeführte Energie oder/und Leistung kann dann durch die Steuervorrichtung aus Signalen ermittelbar sein, welche über die signalübertragungsmäßige Verbindung zur Steuervorrichtung übertragen werden. Die Ermittlung von Energie und Leistung unterscheiden sich dabei lediglich dadurch, dass die Leistung eine zeitbezogene Energieerfassung darstellt.

Zur bedarfsgerechten Anpassung der Übergabe des Atemgases von der Beatmungsvorrichtung an den jeweils zu beatmenden Patienten an die Bedürfnisse des Patienten kann die Atemgasleitungsanordnung an ihrem proximalen Ende eine Kopplungsformation zur Kopplung der Atemgasleitungsanordnung mit einer Atemgas zwischen der Atemgasleitungsanordnung und dem Patienten übertragenden Patientenschnittstelle aufweisen. Eine solche Patientenschnittstelle kann ein Endotrachealtubus, eine Larynxmaske oder ein Kombitubus und dergleichen sein. Die Kopplungsformation kann an einem proximalen Flusssensor ausgebildet sein, welcher am Längsende der Atemgasleitungsanordnung vorgesehen ist, um den Atemgasfluss betragsmäßig zu erfassen.

Selbst wenn die Atemgasleitungsanordnung abschnittsweise oder vollständig beheizbar ist, ist die an die Atemgasleitungsanordnung ankoppelbare Patientenschnittstelle in der Regel nicht beheizbar. Daher kann die Temperatur der Patientenschnittstelle und des Atemgasflusses in der Patientenschnittstelle stärker schwanken als die Temperatur der Atemgasleitungsanordnung und des Atemgasflusses darin.

Unabhängig davon, ob die Patientenschnittstelle ein Tubus oder eine Maske ist, wird stets ein unmittelbar an die Kupplungsformation der Atemgasleitungsanordnung anschließender Abschnitt der Patientenschnittstelle von Umgebungsluft umgeben sein und so durch die Umgebungstemperatur beeinflussbar sein.

Gerade bei der oben beschriebenen Notfall-Transportsituation kann die Umgebungstemperatur erheblich niedriger sein als die Körpertemperatur des Patienten und als eine gewünschte Temperatur des Atemgasflusses. Es kann daher an der Patientenschnittstelle zur Kondensation von Feuchtigkeit aus dem Atemgas kommen, beispielsweise weil an der durch die Umgebungsluft abgekühlten Wand der Patientenschnittstelle die Taupunkttemperatur des Atemgasflusses unterschritten wird. Da die Patientenschnittstelle besonders nah am Patienten angeordnet ist und im Falle eines Trachealtubus sogar in den Körper des Patienten eingeführt ist, ist eine Kondensation von Flüssigkeit im Bereich der Patientenschnittstelle für den zu beatmeten Patienten besonders gefährlich.

Die Kondensation kann an Wänden der Patientenschnittstelle stattfinden, die durch die Umgebungsluft stark abgekühlt sind. Zusätzlich oder alternativ kann die Kondensation als Nebelbildung unmittelbar im Atemgasfluss stattfinden.

Nicht nur ist die Patientenschnittstelle in der Regel unbeheizt, sie ist als Einweg- oder Wegwerf-Artikel in der Regel auch nicht mit Sensoren bestückt. Dies macht die Erkennung und Bekämpfung von Kondensation in der Patientenschnittstelle so schwierig.

Zur Vermeidung einer unerwünschten Kondensation von Feuchtigkeit im Atemgasfluss in der Patientenschnittstelle kann die Steuervorrichtung dazu ausgebildet sein, abhängig von Betriebsparametern des Atemgasflusses, wie beispielsweise Temperatur, Massen- oder Volumenstrom oder/und relative Feuchte, einen Folge-Zustand der Beatmungssituation in Inspirationsrichtung stromabwärts der Kopplungsformation zu ermitteln und abhängig vom Ermittlungsergebnis die Betriebsleistung der Verdampfungsvorrichtung zu verändern. Wird von der Steuervorrichtung ein hinsichtlich Kondensation in der Patientenschnittstelle kritischer Folge-Zustand erkannt, wird die Betriebsleistung der Verdampfungsvorrichtung verändert, in der Regel verringert und so das Risiko einer Kondensation in der Patientenschnittstelle verringert oder sogar beseitigt.

Beispielsweise können für die Ermittlung des Folge-Zustands relevante Betriebsparameter der Beatmungsvorrichtung die Temperatur des Atemgasflusses im proximalen Längsendbereich der Atemgasleitungsanordnung, wie sie vom proximalen Temperatursensor erfasst und an die Steuervorrichtung übertragen wird, und die Umgebungstemperatur umfassen, wie sie vom Umgebungstemperatursensor erfasst und an die Steuervorrichtung übertragen wird. Zusätzlich oder alternativ kann der vom Flusssensor ermittelte Betrag des Atemgasflusses zur Ermittlung des Folge-Zustands herangezogen werden. Ein weiterer relevanter Betriebsparameter kann die Art der Patientenschnittstelle und ihre konstruktive Ausgestaltung sein.

Wiederum kann in einem von der Steuervorrichtung abfragbaren Datenspeicher wenigstens ein, vorzugsweise empirisch ermittelter, Folge-Datenzusammenhang gespeichert sein, welcher einen Satz Betriebsparameter der Beatmungsvorrichtung - dies können mehrere oder alle der oben genannten Betriebsparameter sein - mit einem Folge-Parameter verknüpft, welcher ein Auftreten von Kondensation von Atemgasfeuchtigkeit in der Patientenschnittstelle anzeigt.

Der Folge-Parameter kann ein binärer Parameter sein, welcher entweder einen ersten Wert annehmen kann, der anzeigt, dass keine Kondensation in der Patientenschnittstelle auftritt, oder welcher einen zweiten Wert annehmen kann, der das Gegenteil anzeigt, also dass Kondensation in der Patientenschnittstelle stattfindet.

Abhängig von dem im Datenspeicher hinterlegten Folge-Datenzusammenhang, beispielsweise unter Heranziehung einer analytisch oder numerisch rechnerisch handhabbaren modellhaften Abbildung der Beatmungssituation an der Patientenschnittstelle, kann der Folge-Parameter eine Wahrscheinlichkeit angeben, mit welcher voraussichtlich in der Patientenschnittstelle eine Kondensation von Flüssigkeit aus dem Atemgasfluss stattfindet.

Eine solcher Wahrscheinlichkeitswert kann zusätzlich oder alternativ dadurch zustande kommen, dass bei der empirischen Feststellung des Datenzusammenhangs unter den für die vorliegende Beatmungssituation einschlägigen Betriebsparametern nur in einem Anteil der empirisch untersuchten parametergleichen Fälle Kondensation stattgefunden hat, oder dadurch, dass nicht für alle die vorliegende Beatmungssituation beschreibenden Betriebsparameterwerte ein Datenzusammenhang vorhanden ist, etwa weil der Datenzusammenhang tabellarisch anhand von Stützstellen definiert ist. In diesem Fall können fehlende Werte aus den bestehenden Werten interpoliert oder/und extrapoliert werden. Bei einem komplexen mehrdimensionalen Zusammenhang von Betriebsparametern und einem Folge-Parameter können dabei Unschärfen in der Ermittlung des Folge- Parameters entstehen, die geeignet als Wahrscheinlichkeit wiedergegeben oder interpretiert werden können.

So kann die Steuervorrichtung nach Maßgabe des Folge-Parameters die Betriebsleistung der Verdampfungsvorrichtung verändern, insbesondere verringern, um eine drohende Kondensation in der Patientenschnittstelle zu vermeiden. Beispielsweise kann im Falle des oben genannten einfachen binären Folge-Parameters der eine Wert zu einer Verringerung der Betriebsleistung der Verdampfungsvorrichtung führen, der andere Wert dagegen nicht. Im Falle des beschriebenen Folge-Parameters in Gestalt eines Wahrscheinlichkeitswerts, also eines Werts zwischen 0 und 1 bzw. zwischen 0 % und 100 %, kann ein Schwellen-Wahrscheinlichkeitswert, etwa 40 % oder mehr Wahrscheinlichkeit, einer Kondensation in der Patientenschnittstelle vorbestimmt sein, dessen Überschreiten durch den Folge-Parameter eine Verringerung der Betriebsleistung der Verdampfungsvorrichtung durch Steuervorrichtung zur Folge hat. Dabei kann - mit oder ohne Schwellen-Wahrscheinlichkeitswert - der Betrag der Verringerung der Betriebsleistung vom Betrag der Wahrscheinlichkeit abhängen, mit welcher der Folge-Parameter eine Kondensation in der Patientenschnittstelle in Aussicht stellt. Die Abhängigkeit des Verringerungsbetrags der Betriebsleistung vom Wahrscheinlichkeitsbetrag des Folge-Parameters kann linear sein oder kann progressiv sein, sodass ausgehend von einer vorhandenen Betriebsleistung die Verringerung der Betriebsleistung mit zunehmender Kondensationswahrscheinlichkeit gemäß Folge-Parameter stärker zunimmt als die Kondensationswahrscheinlichkeit.

In einem rechnerisch einfach handzuhabenden und trotzdem hoch effektiven bevorzugten Anwendungsfall kann der Folge-Zustand oder Folge-Parameter der Beatmungssituation eine Folge-Temperatur der Leitungswand der Patientenschnittstelle oder/und des Atemgasflusses in der Patientenschnittstelle umfassen.

Ein erster Vorteil der Verwendung einer Folge-Temperatur zur Beschreibung des Folge-Zustands liegt zum einen darin, dass bei bekanntem konstruktivem Aufbau der Patientenschnittstelle, einschließlich der zur Herstellung der Patientenschnittstelle verwendeten Materialien, und bei bekannten Umgebungs- und Betriebsbedingungen ausgehend vom letzten sensorisch erfassten Betriebszustand etwa im Bereich der Kopplungsformation am proximalen Längsende der Atemgasleitungsanordnung, sich Folge-Temperaturen in Inspirationsrichtung stromabwärts der Kopplungsformation anhand von thermodynamischen Modellen mit guter Genauigkeit berechnen lassen. Somit kann entweder anhand von thermodynamischen Modellen eine Folge-Temperatur in der Patientenschnittstelle ausgehend von, insbesondere den der Patientenschnittstelle nächst gelegenen, messtechnisch erfassten Parametern berechnet werden oder eine Folge-Temperatur kann aufgrund von empirisch erarbeiteten Kennfeldern ausgehend von den genannten Parametern ausgelesen werden oder es kann eine Kombination aus Modellvorhersage und Auslesen aus empirischen Datenzusammenhängen zur Ermittlung der Folge-Temperatur verwendet werden.

Ein zweiter Vorteil der Verwendung einer Folge-Temperatur zur Beschreibung des Folge-Zustands liegt darin, dass allein die Folge-Temperatur ausreichen kann, um eine Kondensation von Flüssigkeit aus dem Atemgasfluss vorherzusagen. Ist die absolute Feuchtigkeit des Atemgasflusses bekannt, sei es durch deren direkte Messung oder sei es durch Messung der relativen Feuchtigkeit und der Temperatur des Atemgasflusses, ist die Taupunkttemperatur des Atemgasflusses bekannt oder ausgehend von hinterlegten Taupunktkurven ermittelbar. Liegt die Folge-Temperatur unter der für den jeweiligen Atemgasfluss ermittelten Taupunkttemperatur, ist eine Kondensation, also ein unerwünschter "rain out", von Feuchtigkeit im Atemgas in der Patientenschnittstelle überwiegend wahrscheinlich.

Wie oben bereits angedeutet, kann die Beatmungsvorrichtung zur Vorhersage einer Folge-Temperatur in der Patientenschnittstelle einen von der Steuervorrichtung abfragbaren Datenspeicher aufweisen, in welchem messtechnisch durch die Beatmungsvorrichtung erfassbare Betriebsparameterwerte, wie etwa die Umgebungstemperatur, oder/und in der Atemgasleitungsanordnung erfassbare Betriebsparameterwerte des Atemgasflusses mit Folge-Temperaturwerten der Leitungswand der Patientenschnittstelle oder/und des Atemgasflusses verknüpft sind. Nur der Vollständigkeit und Klarheit halber sei angemerkt, dass auch ein ausgehend von messtechnisch erfassten Betriebsparameterwerten interpolierter oder extrapolierter Wert als messtechnisch erfasst im Sinne der vorliegenden Anmeldung gilt.

Durch den im genannten Datenspeicher hinterlegten Datenzusammenhang, sei es nun als empirisch ermittelte Datentabelle oder Datenkennfeld oder sei es als analytisches oder numerisches Berechnungsmodell, kann die Steuervorrichtung den Folge-Zustand mit guter Genauigkeit abhängig von der durch den proximalen Temperatursensor erfassten Temperatur des Atemgasflusses im proximalen Längsendbereich der Atemgasleitungsanordnung, von dem vom Flusssensor erfassten Betrag des Atemgasflusses und von der vorgegebenen Soll-Feuchtigkeit des Atemgases ermitteln. So kann durch eine überschaubare Anzahl an bekannten oder messtechnisch erfassbaren Parameterwerten die Wahrscheinlichkeit einer unerwünschten Kondensation in der Patientenschnittstelle mit guter Genauigkeit sehr schnell vorhergesagt werden.

Besonders genau kann die unerwünschte Kondensation von Feuchtigkeit im Atemgas in der Patientenschnittstelle dann vorhergesagt werden, wenn die Steuervorrichtung den Folge-Zustand abhängig von der vom Umgebungstemperatursensor erfassten Umgebungstemperatur ermittelt. Die Umgebungstemperatur, die von außen auf wenigstens einen Abschnitt der Patientenschnittstelle einwirkt, ist ein die Temperatur des strömenden Atemgases in der Patientenschnittstelle und damit eine mögliche Kondensation in der Patientenschnittstelle stark beeinflussender Faktor.

Da es für den Patienten umso nachteiligere Auswirkungen haben kann, je weiter stromabwärts, also je näher am Patienten, die Kondensation im Atemgas in der Patientenschnittstelle stattfindet und damit Flüssigkeit in der Patientenschnittstelle zu befürchten ist, ist gemäß einer vorteilhaften Weiterbildung der vorliegenden Erfindung vorgesehen, dass die Steuervorrichtung den Folge-Zustand für einen dem proximalen Ende der Patientenschnittstelle näher als deren distalem Ende gelegenen Ermittlungsort ermittelt, wobei bevorzugt die Steuervorrichtung den Folge-Zustand für das proximale Ende der Patientenschnittstelle ermittelt.

Zusätzlich oder alternativ kann die Steuervorrichtung den Folge-Zustand für einen Abschnitt der Patientenschnittstelle ermitteln, welcher zwischen der Kopplungsformation und dem Eintrittsort der Patientenschnittstelle in den Körper des Patienten gelegen ist. Dann wird der Folge-Zustand, insbesondere die Folge-Temperatur für genau jenen Abschnitt der Patientenschnittstelle ermittelt, die unmittelbar dem Einfluss der Umgebung und damit der Umgebungstemperatur ausgesetzt ist, die in extremen Betriebssituationen stark von der gewünschten Temperatur des Atemgasflusses abweichen kann.

Zusätzlich kann die Steuervorrichtung dann, wenn ein inspiratorischer Leitungsabschnitt der Atemgasleitungsanordnung durch eine Leitungsheizanordnung beheizbar ist, die Betriebsleistung der Leitungsheizanordnung abhängig vom Ermittlungsergebnis verändern, insbesondere erhöhen. In Abwägung, ob dem Patienten Atemgas kurzfristig geringfügig zu warm oder mit flüssigen Phasenanteilen zugeführt wird, ist die Option der erhöhten Atemgastemperatur zu bevorzugen.

Bevorzugt ist die Kopplungsformation am proximalen Längsende eines Y-Leitungsabschnitts der Atemgasleitungsanordnung ausgebildet, wobei an den beiden distalen Längsenden des Y-Leitungsabschnitts je ein Inspirationsschlauch und ein Exspirationsschlauch angeschlossen sind.

Der Flusssensor kann ein distaler Flusssensor sein, welche im Betriebsgehäuse der Beatmungsvorrichtung aufgenommen ist. Zusätzlich oder alternativ kann der Flusssensor ein proximaler Flusssensor sein, welcher am proximalen Längsende der Atemgasleitungsanordnung, vorzugsweise austauschbar, angeordnet ist. Der proximale Flusssensor kann zwischen der Kopplungsformation und der Patientenschnittstelle angeordnet sein. Ein solcher proximale Flusssensor kann eine hohe Messgenauigkeit bieten und trotzdem kostengünstig sein, wenn er als Differenzdruck-Durchflusssensor ausgebildet ist. Der proximale Flusssensor kann Teil der Kopplungsformation sein und selbst zur Ankopplung der Patientenschnittstelle ausgebildet sein.

Die Verdampfungsvorrichtung weist vorzugsweise eine Heizvorrichtung auf, mit welcher der Flüssigkeitsvorrat erwärmt und verdampft wird. Die Steuervorrichtung ist dann vorzugsweise dazu ausgebildet, die Heizleistung der Heizvorrichtung zu verringern, wenn das oben genannte Ermittlungsergebnis eine Kondensation von Flüssigkeit in der Patientenschnittstelle als sicher oder als überwiegend wahrscheinlich oder als über einer vordefinierten Wahrscheinlichkeitsschwelle oder eine Folge-Temperatur als über einer vordefinierten Folge-Temperaturschwelle liegend angibt.

Alternativ oder zusätzlich kann die Verdampfungsvorrichtung einen Ultraschall-Vernebler aufweisen. Unter diesen Umständen ist die von der Steuervorrichtung abhängig vom Ermittlungsergebnis veränderbare Betriebsleistung die Betriebsleistung des Ultraschall-Verneblers. Die Bedingungen für eine Verringerung der Vernebelungsleistung sind die oben zur Verringerung der Heizleistung genannten Bedingungen.

Die vorliegende Erfindung wird nachfolgend anhand der beiliegenden Zeichnungen näher erläutert werden. Es stellt dar:
- Figur 1: eine schematische Darstellung einer erfindungsgemäßen, zur künstlichen Beatmung eines Patienten hergerichteten Beatmungsvorrichtung, und
- Figur 2: eine grobschematische Darstellung des inspiratorischen Atemgasleitungszweigs der Beatmungsvorrichtung von Figur 1.

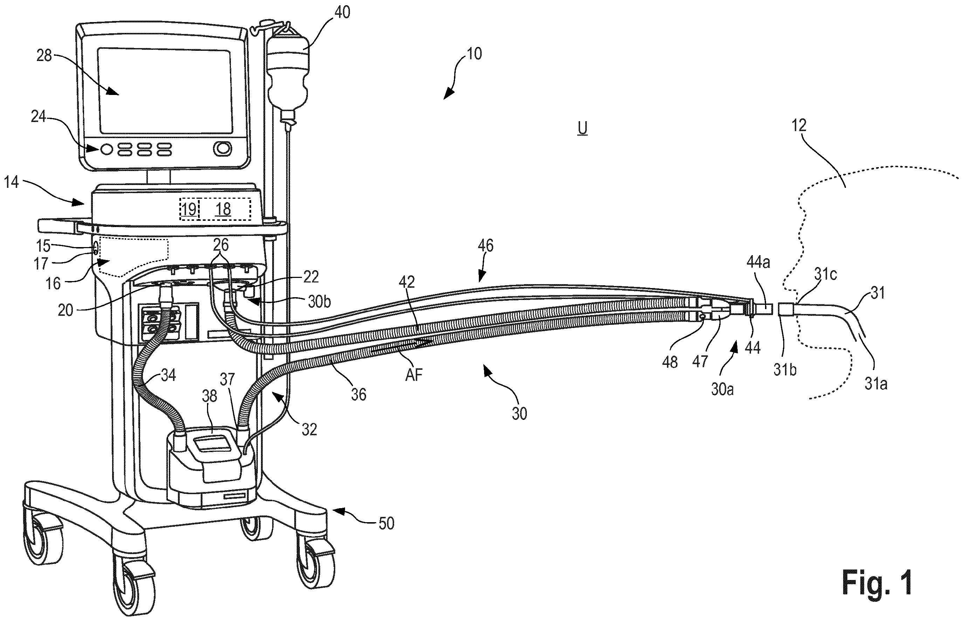

In Figur 1 ist eine erfindungsgemäße Ausführungsform einer Beatmungsvorrichtung allgemein mit 10 bezeichnet. Die Beatmungsvorrichtung 10 dient im dargestellten Beispiel zur künstlichen Beatmung eines humanen Patienten 12.

Die Beatmungsvorrichtung 10 weist ein Gehäuse 14 auf, in welchem eine Ansaugöffnung 15 ausgebildet und - von außen wegen des blickdichten Gehäusematerials nicht erkennbar - eine Strömungsveränderungsvorrichtung 16 sowie eine Steuereinrichtung 18 aufgenommen sind. Die Ansaugöffnung 15 gestattet der Strömungsveränderungsvorrichtung 16, Umgebungsluft aus der Außenumgebung U der Beatmungsvorrichtung anzusaugen und nach an sich bekannter Reinigung durch Filter als Atemgas dem Patienten 12 zuzuführen. Die Ansaugöffnung 15 ist daher eine Atemgas-Quellenanordnung im Sinne der vorliegenden Anmeldung.

In der Ansaugöffnung 15 befindet sich ein Umgebungstemperatursensor 17, welcher die Temperatur der Luft der Umgebung U misst und an die Steuervorrichtung 18 überträgt.

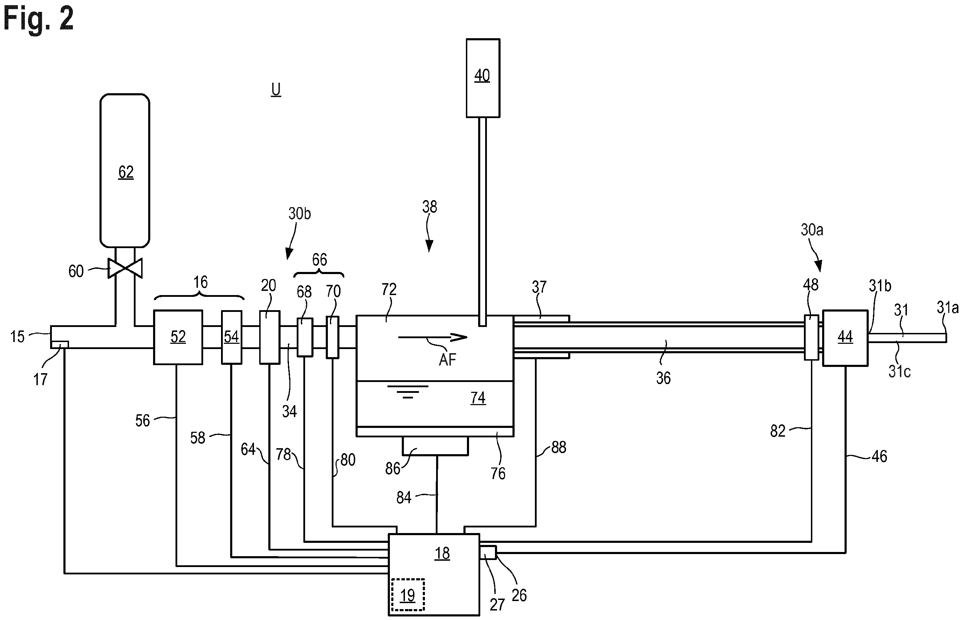

Die Strömungsveränderungsvorrichtung 16 ist in an sich bekannter Weise aufgebaut und kann eine Pumpe, einen Verdichter, ein Gebläse 52, einen Druckbehälter, ein Reduzierventil 54 (s. Fig. 2) und dergleichen aufweisen. Weiter weist die Beatmungsvorrichtung 10 in an sich bekannter Weise ein Inspirationsventil 20 und ein Exspirationsventil 22 auf.

Die Steuereinrichtung 18 ist üblicherweise als Computer oder Mikroprozessor realisiert. Sie umfasst einen in Figur 1 mit 19 bezeichneten Datenspeicher, um für den Betrieb der Beatmungsvorrichtung 10 notwendige Daten speichern und erforderlichenfalls abrufen zu können. Der Datenspeicher 19 kann bei Netzwerkbetrieb auch außerhalb des Gehäuses 14 gelegen und durch eine Datenübertragungsverbindung mit der Steuereinrichtung 18 verbunden sein. Die Datenübertragungsverbindung kann durch eine Kabel- oder eine Funkstrecke gebildet sein. Um jedoch zu verhindern, dass Störungen der Datenübertragungsverbindung sich auf den Betrieb der Beatmungsvorrichtung 10 auswirken können, ist der Datenspeicher 19 bevorzugt in die Steuereinrichtung 18 integriert oder wenigstens im selben Gehäuse 14 wie diese aufgenommen.

Zur Eingabe von Daten in die Beatmungsvorrichtung 10 bzw. genauer in die Steuereinrichtung 18 weist die Beatmungsvorrichtung 10 eine Eingabevorrichtung 24 auf, welche in dem in Figur 1 dargestellten Beispiel durch eine Tastatur repräsentiert ist. Wie weiter unten noch erläutert werden wird, ist die Tastatur nicht der einzige Dateneingang der Steuereinrichtung 18. Tatsächlich kann die Steuereinrichtung 18 Daten über verschiedene Dateneingänge erhalten, etwa über eine Netzwerkleitung, eine Funkstrecke oder über Sensoranschlüsse 26, auf die weiter unten im Einzelnen eingegangen wird.

Zur Ausgabe von Daten an den behandelnden Therapeuten kann die Beatmungsvorrichtung 10 ein Ausgabegerät 28 aufweisen, im dargestellten Beispiel einen Bildschirm.

Zur künstlichen Beatmung ist der Patient 12 mit der Beatmungsvorrichtung 10, genauer mit der Strömungsveränderungsvorrichtung 16 im Gehäuse 14, über eine Atemgasleitungsanordnung 30 verbunden. Der Patient 12 ist hierfür mittels eines Endotrachealtubus als einer Patientenschnittstelle 31 intubiert. Ein proximales Längsende 31a der Patientenschnittstelle 31 gibt den inspiratorischen Atemgasfluss AF in die Lunge des Patienten 12 ab. Durch das proximale Längsende 31a strömt auch der exspiratorische Atemgasstrom in die Atemgasleitungsanordnung ein.

Ein distales Längsende 31b der Patientenschnittstelle 31 ist zur Verbindung mit der Atemgasleitungsanordnung 30 ausgebildet. Ab dem Ort 31c in Inspirationsrichtung stromabwärts bis zum proximalen Längsende 31a ist die Patientenschnittstelle vom Körper des Patienten 12 umgeben. Dies bedeutet im Umkehrschluss, dass die Patientenschnittstelle 31 von seinem distalen Längsende 31b bis zum Ort 31c der Außenumgebung U ausgesetzt ist und mit dieser in, überwiegend konvektiver, Wärmeübertragungsverbindung steht.

Die Atemgasleitungsanordnung 30 weist einen Inspirationsschlauch 32 auf, über welchen frisches Atemgas von der Strömungsveränderungsvorrichtung 16 in die Lunge des Patienten 12 geleitet werden kann. Der Inspirationsschlauch 32 kann unterbrochen sein und einen ersten Inspirationsschlauch 34 und einen zweiten Inspirationsschlauch 36 aufweisen, zwischen welchen eine Befeuchtungsvorrichtung 38 zur gezielten Befeuchtung und gegebenenfalls auch Temperierung des dem Patienten 12 zugeführten inspiratorischen Atemgases vorgesehen sein kann. Die Befeuchtungsvorrichtung 38 kann mit einem externen Flüssigkeitsvorrat 40 verbunden sein, über den Wasser zur Befeuchtung oder auch ein Medikament, etwa zur Entzündungshemmung oder zur Erweiterung der Atemwege, der Befeuchtungsvorrichtung 38 zugeführt werden kann. Bei einem Einsatz der vorliegenden Beatmungsvorrichtung 10 als Anästhesie-Beatmungsvorrichtung können so volatile Anästhetika kontrolliert über die Beatmungsvorrichtung 10 an den Patienten 12 abgegeben werden. Die Befeuchtungsvorrichtung 38 sorgt dafür, dass das frische Atemgas dem Patienten 12 mit einer vorbestimmten Feuchtigkeit, gegebenenfalls unter Zugabe eines Medikamenten-Aerosols, und mit einer vorbestimmten Temperatur zugeführt wird.

Der zweite Inspirationsschlauch 36 ist im vorliegenden Beispiel durch eine Leitungsheizvorrichtung 37 elektrisch beheizbar. Die Leitungsheizvorrichtung 37 ist durch die Steuervorrichtung 18 zum Betrieb ansteuerbar. Abweichend vom Gesagten kann auch der erste Inspirationsschlauch 34 beheizbar sein oder/und kann der wenigstens eine Schlauch 34 oder/und 36 durch eine andere als eine elektrische Leitungsheizvorrichtung 37 beheizbar sein, etwa durch Umspülung mit einem Wärmetauschmedium.

Die Atemgasleitungsanordnung 30 weist neben dem bereits erwähnten Inspirationsventil 20 und Exspirationsventil 22 weiter einen Exspirationsschlauch 42 auf, über welchen verstoffwechseltes Atemgas aus der Lunge des Patienten 12 in die Außenumgebung U abgeblasen wird.

Am distalen Längsende 30b der Atemgasleitungsanordnung 30 sind der Inspirationsschlauch 32 mit dem Inspirationsventil 20 und der Exspirationsschlauch 42 mit dem Exspirationsventil 22 gekoppelt. Von den beiden Ventilen ist vorzugsweise jeweils nur eines gleichzeitig zum Durchlass einer Gasströmung geöffnet. Die Betätigungssteuerung der Ventile 20 und 22 erfolgt ebenfalls durch die Steuereinrichtung 18.

Während eines Beatmungszyklus ist zunächst für die Dauer der Inspirationsphase das Exspirationsventil 22 geschlossen und das Inspirationsventil 20 geöffnet, sodass frisches inspiratorisches Atemgas vom Gehäuse 14 zum Patienten 12 geleitet werden kann. Eine Strömung des frischen Atemgases wird durch gezielte Druckerhöhung des Atemgases durch die Strömungsveränderungsvorrichtung 16 bewirkt. Aufgrund der Druckerhöhung strömt das frische Atemgas in die Lunge des Patienten 12 und expandiert dort den lungennahen Körperbereich, also insbesondere den Brustkorb, gegen die individuelle Elastizität der lungennahen Körperteile. Hierdurch steigt auch der Gasdruck im Inneren der Lunge des Patienten 12 an.

Am Ende der Inspirationsphase wird das Inspirationsventil 20 geschlossen und das Exspirationsventil 22 geöffnet. Es beginnt die Exspirationsphase. Aufgrund des bis zum Ende der Inspirationsphase erhöhten Gasdrucks des in der Lunge des Patienten 12 befindlichen Atemgases strömt dieses nach dem Öffnen des Exspirationsventils 22 in die Außenumgebung U, wobei sich mit fortschreitender Strömungsdauer der Gasdruck in der Lunge des Patienten 12 verringert. Erreicht der Gasdruck in der Lunge 12 einen an der Beatmungsvorrichtung 10 eingestellten positiven end-exspiratorischen Druck (PEEP), also einen geringfügig höheren Druck als den Atmosphärendruck, wird die Exspirationsphase mit dem Schließen des Exspirationsventils 22 beendet und es schließt sich ein weiterer Beatmungszyklus an.

Während der Inspirationsphase wird dem Patienten 12 das sogenannte Beatmungs-Tidalvolumen zugeführt, also das Atemgas-Volumen pro Atemhub. Das Beatmungs-Tidalvolumen multipliziert mit der Anzahl an Beatmungszyklen pro Minute, also multipliziert mit der Beatmungsfrequenz, ergibt das Minutenvolumen der vorliegend durchgeführten künstlichen Beatmung.

Bevorzugt ist die Beatmungsvorrichtung 10, insbesondere die Steuereinrichtung 18, dazu ausgebildet, Beatmungs-Betriebsparameter, die den Beatmungsbetrieb der Beatmungsvorrichtung 10 kennzeichnen, während des Beatmungsbetriebs wiederholt zu aktualisieren bzw. zu ermitteln, um sicherzustellen, dass der Beatmungsbetrieb zu jedem Zeitpunkt möglichst optimal auf den jeweils zu beatmenden Patienten 12 abgestimmt ist. Besonders vorteilhaft erfolgt die Bestimmung eines oder mehrerer Beatmungs-Betriebsparameter mit der Beatmungsfrequenz, sodass für jeden Beatmungszyklus aktuelle und damit optimal an den Patienten 12 angepasste Beatmungs-Betriebsparameter bereitgestellt werden können.

Hierzu kann die Beatmungsvorrichtung 10 mit einem oder mehreren Sensoren datenübertragungsmäßig verbunden sein, welche den Zustand des Patienten oder/und den Betrieb der Beatmungsvorrichtung 10 überwachen. Lediglich beispielhaft für eine Reihe möglicher Sensoren sei in Figur 1 ein proximaler Flusssensor 44 genannt, welcher den in der Atemgasleitungsanordnung 30 herrschenden Atemgasfluss betragsmäßig erfasst. Der vorzugsweise als Differenzdrucksensor ausgebildete proximale Flusssensor 44 kann mittels einer Sensorleitungsanordnung 46 mit den Dateneingängen 26 der Steuereinrichtung 18 gekoppelt sein. Die Sensorleitungsanordnung 46 kann, muss jedoch nicht, elektrische Signalübertragungsleitungen umfassen. Sie kann ebenso Schlauchleitungen aufweisen, die den in Strömungsrichtung beiderseits des Flusssensors 44 herrschenden Gasdruck an die Dateneingänge 26 übertragen, wo diese von nur in Figur 2 dargestellten Drucksensoren 27 quantifiziert werden.

Genauer weist die Atemgasleitungsanordnung 30 im bevorzugten Ausführungsbeispiel an ihrem proximalen Längsendbereich 30a einen gesondert ausgebildeten Y-Leitungsabschnitt 47 auf, welcher an seinem distalen Endbereich mit dem zweiten Inspirationsschlauch 36 und dem Exspirationsschlauch 42 verbunden ist und welcher an seinem proximalen Endbereich mit dem proximale Flusssensor 44 verbunden ist.

Der proximale Flusssensor 44 weist an seinem proximalen Endbereich eine Kopplungsformation 44a auf, mit welcher die Patientenschnittstelle 31, die anstelle eines Tubus auch eine Maske sein könnte, mit dem proximalen Flusssensor 44 und folglich mit der Atemgasleitungsanordnung 30 koppelbar ist.

Der zweite Inspirationsschlauch 36 weist an seinem proximalen Längsendbereich einen proximalen Temperatursensor 48 auf, welcher die Temperatur des Atemgasflusses AF im zweiten Inspirationsschlauch 36 möglichst nah am Patienten 12 misst und an die Steuervorrichtung 18 überträgt.

Lediglich der Vollständigkeit halber sei darauf hingewiesen, dass die erfindungsgemäße Beatmungsvorrichtung 10 als mobile Beatmungsvorrichtung 10 auf einem rollbaren Gestell 50 aufgenommen sein kann.

In Figur 2 ist eine grobschematische Ansicht des inspiratorischen Leitungszweigs der Beatmungsvorrichtung 10 von Figur 1 gezeigt.

Die Strömungsveränderungsvorrichtung 16 umfasst ein Gebläse 52 und ein in Inspirationsrichtung stromabwärts desselben gelegenes Reduzierventil 54. Es kann auch nur das Gebläse 52 vorgesehen sein. Die Steuervorrichtung 18 kann das Gebläse 52 und das Reduzierventil 54 über Leitungen 56 bzw. 58 zum Betrieb ansteuern.

Das Gebläse 52 kann durch die Ansaugöffnung 15 Umgebungsluft aus der Außenumgebung U ansaugen. Zusätzlich oder alternativ kann über ein Ventil 60 Gas, etwa reiner Sauerstoff, aus einem Vorratsbehälter 62 als Atemgas verwendet oder der angesaugten Umgebungsluft beigemischt werden. Daher bilden im dargestellten Ausführungsbeispiel die Ansaugöffnung 15 und der Vorratsbehälter 62 gemeinsam eine Atemgas-Quellenanordnung.

Die angesaugte Umgebungsluft wird in der Beatmungsvorrichtung 10 in nicht dargestellten Filtern in an sich bekannter Weise gereinigt.

In Inspirationsrichtung stromabwärts der Strömungsveränderungsvorrichtung 16 befindet sich das Inspirationsventil 20, welches über eine Leitung 64 von der Steuervorrichtung 18 zum Öffnen und Schließen steuerbar ist.

In Inspirationsrichtung weiter stromabwärts schließt sich an das Inspirationsventil 20 der erste Inspirationsschlauch 34 an.

Die Beatmungsvorrichtung 10 umfasst in Inspirationsrichtung stromaufwärts der Beatmungsvorrichtung 38 in engerem Sinne eine Feuchte-Sensoranordnung 66, welche im wirtschaftlich bevorzugten dargestellten Fall einen Sensor 68 zur Ermittlung der relativen Feuchte des inspiratorischen Atemgasflusses AF und einen Temperatursensor 70 zur Ermittlung der Atemgastemperatur im Bereich der Feuchteerfassung durch den Sensor 68 umfasst. Alternativ könnte die Feuchte-Sensoranordnung 66 nur einen Sensor zur Erfassung der absoluten Feuchtigkeit des Atemgases umfassen, der jedoch aus Kostengründen nicht bevorzugt ist.

Die Feuchte-Sensoranordnung 66 erfasst mit der relativen Feuchtigkeit und der Temperatur des Atemgases stromaufwärts der Befeuchtungsvorrichtung 38 die relative Feuchtigkeit und die Temperatur des Atemgases nach den Atemgasquellen 15 und 62, aber vor Einleitung von Befeuchtungsmaßnahmen durch die Beatmungsvorrichtung 10. Die Feuchte-Sensoranordnung 66 kann in dem in Figur 1 gezeigten Gehäuse der Befeuchtungsvorrichtung 38 angeordnet sein, jedoch stromaufwärts der Befeuchtungsvorrichtung 38, welche gebildet ist durch eine Befeuchtungskammer 72 mit einem Flüssigkeitsvorrat 74, dem über eine thermische Verdampfungsvorrichtung 76 in Gestalt einer Heizplatte thermische Energie zur Verdampfung von Flüssigkeit zuführbar ist.

Die Erfassungswerte der Sensoren 68 und 70 sind durch Leitungen 78 und 80 an die Steuervorrichtung 18 übertragbar.

Die Steuervorrichtung 18 kann aus den so erhaltenen Erfassungswerten für die relative Feuchtigkeit und die Temperatur des Atemgases vor Erreichen der Befeuchtungsvorrichtung 38 die absolute Feuchtigkeit des Atemgases ermitteln und mit einer gewünschten Soll-Feuchtigkeit des Atemgases vergleichen.

Die Soll-Feuchtigkeit des Atemgases kann sich beispielsweise aus einem im Datenspeicher 19 hinterlegten Datenzusammenhang aus Signalen des proximalen Temperatursensors 48 und gegebenenfalls auch des vom proximalen Flusssensor 44 erfassten proximalen Atemgasflusses ergeben. Alternativ kann eine gewünschte Soll-Feuchtigkeit des Atemgases auch manuell über die Eingabevorrichtung 24 eingegeben werden.

Ausgehend von der Soll-Feuchtigkeit des Atemgases sowie weiter abhängig von den Signalen des proximalen Temperatursensors 48, welche über eine Leitung 82 an die Steuervorrichtung 18 übertragen werden, der Feuchte-Sensoranordnung 66 und des proximalen Flusssensors 44 ermittelt die Steuervorrichtung 18 eine der Verdampfungsvorrichtung 76 in Gestalt einer Heizplatte zuzuführende Betriebsleistung. Über eine Leitung 84 steuert die Steuervorrichtung 18 eine Energieversorgung 86 der Verdampfungsvorrichtung 76 entsprechend der ermittelten Betriebsleistung an. Über die Leitung 84 erhält die Steuervorrichtung 18 Informationen über die an die Verdampfungsvorrichtung 76 abgegebene Betriebsleistung, beispielsweise in Gestalt der an der Verdampfungsvorrichtung 76 anliegenden Spannung und der dieser zufließenden Stromstärke. Die Energieversorgungsvorrichtung 86 kann beispielsweise durch Pulsbreitenmodulation eine variable Leistung an die Verdampfungsvorrichtung 76 abgeben. Bei Pulsbreitenmodulation ist die an der Verdampfungsvorrichtung 76 anliegende Spannung eine zeitlich gemittelte Spannung.

Durch die Verdampfungsvorrichtung 76 wird Flüssigkeit aus dem Flüssigkeitsvorrat 74 verdampft und durch den den Flüssigkeitsvorrat 74 überströmenden Atemgasfluss AF mitgeführt.

Um Wärmeverluste der Verdampfungsvorrichtung 76 an die Umgebung U zu berücksichtigen, ermittelt die Steuervorrichtung 18 die der Verdampfungsvorrichtung 76 über die Energieversorgungsvorrichtung 86 zuzuführende Energie pro Zeit auch unter Berücksichtigung der durch den Umgebungstemperatursensor 17 ermittelten Umgebungstemperatur.

Zur Ermittlung der der Verdampfungsvorrichtung 76 abhängig von der Soll-Feuchtigkeit des Atemgases und weiter abhängig von der proximalen Atemgastemperatur, der durch die Feuchte-Sensoranordnung 66 mittelbar erfassten absoluten Feuchtigkeit, des durch den proximalen Flusssensor 44 erfassten proximalen Atemgasflusses und weiter abhängig von der Umgebungstemperatur zuzuführenden Betriebsleistung ist im Datenspeicher 19 ein entsprechender Datenzusammenhang hinterlegt, welcher vorab im Labor durch Parametervariation unter kontrollierten Bedingungen für eine baugleiche Beatmungsvorrichtung 10 bestimmt wurde.

Es ist daher davon auszugehen, dass durch den Betrieb der Verdampfungsvorrichtung 76, gesteuert durch die Steuervorrichtung 18 nach Maßgabe der genannten Parameter, der inspiratorische Atemgasfluss AF die Befeuchtungsvorrichtung 38 mit dem Soll-Feuchtigkeitsgehalt verlässt. Der inspiratorische Atemgasfluss tritt dann in den zweiten Inspirationsschlauch 36 ein, der durch die Leitungsheizvorrichtung 37 beheizbar ist. Der Betrieb der Leitungsheizvorrichtung 37 ist durch die Steuervorrichtung 18 mittels einer Leitung 88 nach Maßgabe der vom proximalen Temperatursensor 48 erfassten Temperatur derart steuerbar, dass die vom proximalen Temperatursensor 48 erfasste proximale Atemgastemperatur über der Taupunkttemperatur des befeuchteten inspiratorischen Atemgasflusses AF liegt.

Die Taupunkttemperatur kann ausgehend von der Annahme, dass das Atemgas nach Durchgang durch die Befeuchtungsvorrichtung 38 die Soll-Feuchtigkeit aufweist, anhand der vom proximalen Temperatursensor 48 erfassten proximalen Atemgastemperatur und weiter anhand einer im Datenspeicher 19 hinterlegten Taupunkttemperaturkurve ermittelt werden. Somit ergibt sich aus der Taupunkttemperaturkurve eine Soll-Temperatur, die am proximalen Temperatursensor 48 nicht unterschritten werden sollte, vorzugsweise sogar mit gewissem Sicherheitsabstand nicht unterschritten werden sollte.

Mit Hinterlegung einer Taupunkttemperaturkurve im Datenspeicher 19 ist nicht notwendigerweise die Hinterlegung einer stetig differenzierbaren Kurve gemeint. Es reicht aus, die Taupunkttemperaturkurve durch ausreichend zahlreiche Stützstellen angenähert zu hinterlegen, beispielsweise als Tabelle. Ebenso kann für die Taupunkttemperatur eine angenäherte mathematische Funktion hinterlegt sein, mit welcher eine Taupunkttemperatur ausgehend von einer bekannten absoluten Feuchtigkeit oder einem bekannten Wertepaar aus relativer Feuchtigkeit und zugeordneter Temperatur ermittelt werden kann.

Ein Problem einer ordnungsgemäßen Versorgung des Patienten mit ausreichend befeuchtetem, aber nicht überbefeuchtetem Atemgas liegt in der Patientenschnittstelle 31. Die Patientenschnittstelle 31 ist weder beheizbar, wie der zweite Inspirationsschlauch 36, noch mit irgendwelchen Sensoren versehen, die den Zustand der Beatmung in der Patientenschnittstelle 31 erfassen könnten. Dies liegt in dem üblichen Charakter der Patientenschnittstelle 31 als Wegwerf- oder Einweg-Schnittstelle begründet.

Gerade dann, wenn die Umgebungstemperaturen sehr niedrig sind, wie etwa bei Notfall-Einsätzen, wird daher wenigstens der der Außenumgebung U ausgesetzte Abschnitt zwischen dem distalen Längsende 31b der Patientenschnittstelle 31 und dem Eintritt der Patientenschnittstelle 31 in den Patienten 12 am Ort 31c stark von der Umgebungstemperatur beeinflusst. Dabei kann die Patientenschnittstelle 31 in diesem Abschnitt so stark durch die Umgebungstemperatur abgekühlt werden, dass es unerwünschterweise in der Patientenschnittstelle 31 zu einem "rain out" kommt. Dieses unerwünschte Kondensieren von Flüssigkeit aus dem inspiratorischen Atemgasfluss AF wird in der Regel am ehesten an der durch die Umgebungsluft abgekühlten Leitungswand der Patientenschnittstelle 31 erfolgen. Es ist jedoch auch nicht ausgeschlossen, dass der Atemgasstrom AF in der Patientenschnittstelle 31 unter dem Einfluss der Umgebungstemperatur so weit abkühlt, dass es zu Nebelbildung im Atemgasfluss AF kommt.

Um eine solche unerwünschte Kondensation von Flüssigkeit in der Patientenschnittstelle 31 zu vermeiden, ist die Steuervorrichtung 18 dazu ausgebildet, ausgehend von an sie durch die zur Verfügung stehenden Sensoren übermittelten Betriebsparametern der Beatmungsvorrichtung und insbesondere des inspiratorischen Atemgasflusses AF einen sich unter Einfluss der Umgebungstemperatur ergebenden Folge-Zustand der Beatmungssituation in der Patientenschnittstelle 31 in Inspirationsrichtung stromabwärts der Kopplungsformation 44a zu ermitteln und ausgehend von diesem ermittelten Folge-Zustand zu bestimmen, ob in der Patientenschnittstelle 31 Kondensation zu erwarten ist.

Hierzu können im Datenspeicher 19 entweder empirisch ermittelte Datenzusammenhänge oder ein analytisch oder numerisch lösbares Gleichungssystem als Modellbeschreibung des thermischen Zustands der Patientenschnittstelle 31 im Betrieb abhängig von Betriebsdaten der Beatmungsvorrichtung 10 und ihrer Umgebung U hinterlegt sein.

In einem besonders einfachen und schnell ermittelbaren Rechenmodell ist der Folge-Zustand der Patientenschnittstelle 31 eine Folge-Temperatur der Patientenschnittstelle 31, welche beispielsweise ausgehend von der proximalen Atemgastemperatur am Temperatursensor 48 unter weiterer Berücksichtigung der konstruktiven Ausgestaltung der Patientenschnittstelle 31 einschließlich der darin verwendeten Materialien sowie unter Berücksichtigung der Umgebungstemperatur, gemessen durch den Umgebungstemperatursensor 17, ermittelbar ist. Zur Ermittlung der Folge-Temperatur kann außerdem der vom proximalen Flusssensor 44 erfasste inspiratorische Atemgasfluss AF und gewünschtenfalls sogar ein ebenfalls vom proximalen Flusssensor 44 erfasster exspiratorischer Atemgasfluss herangezogen werden

So kann die Steuervorrichtung 18, die aufgrund der oben beschriebenen Steuerung der Beatmungsvorrichtung 10 bereits über die Taupunkttemperatur des inspiratorischen Atemgasflusses AF verfügt, dann von einer unmittelbar drohenden Kondensationsgefahr in der Patientenschnittstelle 31 ausgehen, wenn eine aufgrund von empirisch ermitteltem Datenzusammenhang oder/und aufgrund eines thermodynamischen Modells ermittelte Folge-Temperatur der Patientenschnittstelle 31 in einem in Inspirationsrichtung stromaufwärts des distalen Längsendes 31b gelegenen Punkt die Taupunkttemperatur unterschreitet. Zur Sicherheit des Patienten wird bevorzugt nicht unmittelbar die Taupunkttemperatur, sondern eine Schwellentemperatur herangezogen, welche eine um eine vorbestimmte Sicherheitsmarge erhöhte Taupunkttemperatur ist.

Für den Fall, dass die Steuervorrichtung 18 auf diese Art und Weise ermittelt, dass Kondensation in der Patientenschnittstelle 31 mit Sicherheit oder mit überwiegender Wahrscheinlichkeit oder mit mehr als einer vorbestimmten Grenz-Wahrscheinlichkeit auftritt, ist die Steuervorrichtung 18 dazu ausgebildet, die der Verdampfungsvorrichtung 76 zugeführte Betriebsleistung zu verringern. Der Verringerungsbetrag der Betriebsleistung der Verdampfungsvorrichtung 76 kann dabei umso höher gewählt sein, je größer die Kondensationsgefahr aufgrund des Ermittlungsergebnisses ist, beispielsweise je stärker die Folge-Temperatur der Patientenschnittstelle 31 die Schwellentemperatur oder sogar die Taupunkttemperatur betragsmäßig überschreitet.

Zusätzlich kann die Steuervorrichtung 18 auch die Betriebsleistung der Leitungsheizanordnung 37 erhöhen, um die Temperatur des inspiratorischen Atemgasflusses AF auf dem Weg zum proximalen Längsende 30a der Atemgasleitungsanordnung 30 zu erhöhen. Da jedoch die vorliegende Steuervorrichtung 18 auch für nicht beheizbare Inspirationsschläuche eine Kondensation von Atemgas in der Patientenschnittstelle 31 verhindern können soll, ist auf jeden Fall die Verringerung der Betriebsleistung der Verdampfungsvorrichtung 76 implementiert.

## Patentansprüche

1. Beatmungsvorrichtung (10) zur künstlichen Beatmung eines Patienten (12), umfassend:
- eine Atemgas-Quellenanordnung (15, 62), welche ein inspiratorisches Atemgas zur künstlichen Beatmung des Patienten (12) bereitstellt,
- eine Strömungsveränderungsvorrichtung (16), welche dazu ausgebildet ist, einen inspiratorischen Atemgasfluss (AF) zu erzeugen und betragsmäßig zu verändern,
- eine Befeuchtungsvorrichtung (38), welche dazu ausgebildet ist, die absolute Feuchtigkeit des inspiratorischen Atemgasflusses (AF) betragsmäßig zu erhöhen, wobei die Befeuchtungsvorrichtung (38) hierzu einen Flüssigkeitsvorrat (40) und eine leistungsveränderliche Verdampfungsvorrichtung (76) aufweist,
- eine Atemgasleitungsanordnung (30) mit einem im Betrieb dem Patienten (12) näher gelegenen proximalen Längsende (30a) und mit einem im Betrieb vom Patienten weiter entfernt gelegenen distalen Längsende (30b), um den inspiratorischen Atemgasfluss (AF) von der Befeuchtungsvorrichtung (38) zum Patienten (12) hin zu fördern,
- einen proximalen Temperatursensor (48), welcher dazu ausgebildet ist, die Temperatur des Atemgasflusses (AF) im proximalen Längsendbereich (30a) der Atemgasleitungsanordnung (30) zu erfassen,
- eine Feuchte-Sensoranordnung (66), welche dazu ausgebildet ist, wenigstens einen Feuchte-Zustandswert des inspiratorischen Atemgasflusses (AF) zu erfassen, der mittelbar oder unmittelbar eine absolute Feuchtigkeit des inspiratorischen Atemgasflusses (AF) repräsentiert,
- einen Flusssensor (44), welcher dazu ausgebildet ist, den Atemgasfluss (AF) betragsmäßig zu erfassen,
- eine Steuervorrichtung (18), welche signalübertragungsmäßig mit dem proximalen Temperatursensor (48), der Feuchte-Sensoranordnung (66) und dem Flusssensor (44) verbunden ist und welche dazu ausgebildet ist, die Betriebsleistung der Verdampfungsvorrichtung (76) abhängig von einer vorgegebenen Soll-Feuchtigkeit des Atemgases und abhängig von Signalen des proximalen Temperatursensors (48), der Feuchte-Sensoranordnung (66) und des Flusssensors (44) zu steuern,
wobei die Feuchte-Sensoranordnung (66) in Inspirationsrichtung des Atemgasflusses (AF) stromaufwärts der Befeuchtungsvorrichtung (38) angeordnet ist und dazu ausgebildet ist, den Feuchte-Zustandswert stromaufwärts der Befeuchtungsvorrichtung (38)zu erfassen, wobei die Beatmungsvorrichtung (10) wenigstens einen Umgebungstemperatursensor (17) aufweist, welcher dazu ausgebildet ist, die Temperatur der Umgebung (U) der Beatmungsvorrichtung (10) zu erfassen, wobei die Steuervorrichtung (18) signalübertragungsmäßig mit dem wenigstens einen Umgebungstemperatursensor (17) verbunden ist und dazu ausgebildet ist, die Betriebsleistung der Verdampfungsvorrichtung (76) zusätzlich zu den bereits genannten Abhängigkeiten auch abhängig von Signalen des wenigstens einen Umgebungstemperatursensors (17) zu steuern.

2. Beatmungsvorrichtung (10) nach Anspruch 1,
**dadurch gekennzeichnet, dass** sie eine Eingabevorrichtung (24) zur Eingabe einer Soll-Temperatur des Atemgasflusses (AF) im proximalen Längsendbereich (30a) der Atemgasleitungsanordnung (30) oder/und zur Eingabe einer Soll-Feuchtigkeit des Atemgases oder/und zur Eingabe eines Soll-Atemgasflusses aufweist.

3. Beatmungsvorrichtung (10) nach Anspruch 2,
**dadurch gekennzeichnet, dass** sie eine Eingabevorrichtung (24) zur Eingabe einer Soll-Temperatur des Atemgasflusses im proximalen Längsendbereich (30a) der Atemgasleitungsanordnung (30) aufweist und einen von der Steuervorrichtung (18) abfragbaren Datenspeicher (19) aufweist, in welchem ein Zusammenhang zwischen Temperaturwerten des Atemgasflusses (AF) im proximalen Längsendbereich (30a) der Atemgasleitungsanordnung (30) und zugeordneten Soll-Feuchtigkeitswerten des Atemgases gespeichert ist.

4. Beatmungsvorrichtung (10) nach einem der vorhergehenden Ansprüche,
**dadurch gekennzeichnet, dass** wenigstens ein inspiratorischer Leitungsabschnitt (36) der Atemgasleitungsanordnung (30) eine Leitungsheizvorrichtung (37) aufweist, mit welcher der inspiratorische Leitungsabschnitt (36) beheizbar ist, wobei bevorzugt die Steuervorrichtung (18) dazu ausgebildet ist, die Betriebsleistung der Leitungsheizvorrichtung (37) abhängig von einer vorgegebenen Soll-Temperatur des Atemgasflusses (AF) im proximalen Längsendbereich (30a) der Atemgasleitungsanordnung (30) zu steuern.

5. Beatmungsvorrichtung (10) nach einem der vorhergehenden Ansprüche,
**dadurch gekennzeichnet, dass** die Feuchte-Sensoranordnung (66) in Inspirationsrichtung des Atemgasflusses (AF) stromabwärts der Atemgas-Quellenanordnung (15, 62) angeordnet ist.

6. Beatmungsvorrichtung (10) nach einem der vorhergehenden Ansprüche,
**dadurch gekennzeichnet, dass** die Steuervorrichtung (18) mit der Verdampfungsvorrichtung (76) oder/und mit einer Energieversorgung derselben oder/und mit wenigstens einem Energiesensor, welcher dazu ausgebildet ist, eine der Verdampfungsvorrichtung (76) zugeführte Energie zu erfassen, derart signalübertragungsmäßig verbunden ist, dass die der Verdampfungsvorrichtung (76) aktuell zugeführte Energie oder/und Leistung durch die Steuervorrichtung (18) aus Signalen ermittelbar ist, welche über die signalübertragungsmäßige Verbindung übertragen werden.

7. Beatmungsvorrichtung (10) nach einem der vorhergehenden Ansprüche,
**dadurch gekennzeichnet, dass** die Atemgasleitungsanordnung (30) an ihrem proximalen Ende (30a) eine Kopplungsformation (44a) zur Kopplung der Atemgasleitungsanordnung (30) mit einer Atemgas zwischen der Atemgasleitungsanordnung (30) und dem Patienten (12) übertragenden Patientenschnittstelle (31), wie etwa Endotrachealtubus, Larynxmaske oder Kombitubus und dergleichen, aufweist, wobei die Steuervorrichtung (18) dazu ausgebildet ist, abhängig von Betriebsparametern der Beatmungsvorrichtung (10), insbesondere des Atemgasflusses (AF), einen Folge-Zustand der Beatmungssituation in Inspirationsrichtung stromabwärts der Kopplungsformation (44a) zu ermitteln und abhängig vom Ermittlungsergebnis die Betriebsleistung der Verdampfungsvorrichtung (76) zu verändern.

8. Beatmungsvorrichtung (10) nach Anspruch 7,
**dadurch gekennzeichnet, dass** der Folge-Zustand der Beatmungssituation eine Folge-Temperatur der Leitungswand der Patientenschnittstelle (31) oder/und des Atemgasflusses (AF) umfasst.

9. Beatmungsvorrichtung (10) nach Anspruch 8,
**dadurch gekennzeichnet, dass** sie einen von der Steuervorrichtung (18) abfragbaren Datenspeicher (19) aufweist, in welchem Betriebsparameterwerte des Atemgasflusses (AF) mit Folge-Temperaturwerten der Leitungswand der Patientenschnittstelle (31) oder/und des Atemgasflusses (AF) verknüpft sind.

10. Beatmungsvorrichtung (10) nach einem der Ansprüche 7 bis 9,
**dadurch gekennzeichnet, dass** die Steuervorrichtung (18) den Folge-Zustand abhängig von der durch den proximalen Temperatursensor (48) erfassten Temperatur des Atemgasflusses (AF) im proximalen Längsendbereich (30a) der Atemgasleitungsanordnung (30), von dem vom Flusssensor (44) erfassten Betrag des Atemgasflusses (AF) und von der vorgegebenen Soll-Feuchtigkeit des Atemgases ermittelt.

11. Beatmungsvorrichtung (10) nach einem der Ansprüche 7 bis 10,
**dadurch gekennzeichnet, dass** die Steuervorrichtung (18) den Folge-Zustand abhängig von der vom Umgebungstemperatursensor (17) erfassten Umgebungstemperatur ermittelt.

12. Beatmungsvorrichtung (10) nach einem der Ansprüche 7 bis 11,
**dadurch gekennzeichnet, dass** die Steuervorrichtung (18) den Folge-Zustand für einen dem proximalen Ende (31a) der Patientenschnittstelle (31) näher als deren distalem Ende (31b) gelegenen Ermittlungsort ermittelt, wobei bevorzugt die Steuervorrichtung (18) den Folge-Zustand für das proximale Ende (31a) der Patientenschnittstelle (31) ermittelt.

13. Beatmungsvorrichtung (10) nach einem der Ansprüche 7 bis 11,
**dadurch gekennzeichnet, dass** die Steuervorrichtung (18) den Folge-Zustand für einen Abschnitt der Patientenschnittstelle (31) ermittelt, welcher zwischen der Kopplungsformation (44a) und dem Eintrittsort (31c) der Patientenschnittstelle (31) in den Körper des Patienten (12) gelegen ist.

14. Beatmungsvorrichtung (10) nach einem der Ansprüche 7 bis 13, unter Einbeziehung des Anspruchs 4,
**dadurch gekennzeichnet, dass** die Steuervorrichtung dazu ausgebildet ist, abhängig vom Ermittlungsergebnis die Temperatur des Atemgases am proximalen Temperatursensor (48) und hierzu die Betriebsleistung der Leitungsheizvorrichtung (37) zu verändern.

## Claims

1. A ventilation apparatus (10) for artificial ventilation of a patient (12), encompassing:
- a respiratory gas source arrangement (15, 62) that furnishes an inspiratory respiratory gas for artificial ventilation of the patient (12);
- a flow modification apparatus (16) that is embodied to generate and to quantitatively modify an inspiratory respiratory gas flow (AF);
- a humidification apparatus (38) that is embodied to quantitatively increase the absolute moisture content of the inspiratory respiratory gas flow (AF), the humidification apparatus (38) comprising for that purpose a liquid reservoir (40) and a modifiable-power-level evaporation apparatus (76);
- a respiratory gas conduit arrangement (30) having a proximal longitudinal end (30a) located closer to the patient (12) during operation and having a distal longitudinal end (30b) located farther from the patient during operation, in order to convey the inspiratory respiratory gas flow (AF) from the humidification apparatus (38) to the patient (12);
- a proximal temperature sensor (48) that is embodied to detect the temperature of the respiratory gas flow (AF) in the proximal longitudinal end region (30a) of the respiratory gas conduit arrangement (30);
- a humidity sensor arrangement (66) that is embodied to detect at least one humidity state value of the inspiratory respiratory gas flow (AF) which indirectly or directly represents an absolute moisture content of the inspiratory respiratory gas flow (AF);
- a flow sensor (44) that is embodied to quantitatively detect the respiratory gas flow (AF);
- a control apparatus (18) which is signal-transferringly connected to the proximal temperature sensor (48), to the humidity sensor arrangement (66), and to the flow sensor (44), and which is embodied to control the operating power level of the evaporation apparatus (76) depending on a predefined setpoint moisture content of the respiratory gas and depending on signals of the proximal temperature sensor (48), of the humidity sensor arrangement (66), and of the flow sensor (44),
the humidity sensor arrangement (66) being arranged upstream, in an inspiration direction of the respiratory gas flow (AF), from the humidification apparatus (38) and being embodied to detect the humidity state value upstream from the humidification apparatus (38); the ventilation apparatus (10) comprising at least one ambient temperature sensor (17) that is embodied to detect the temperature of the environment (U) of the ventilation apparatus (10); the control apparatus (18) being signal-transferringly connected to the at least one ambient temperature sensor (17) and being embodied to control the operating power level of the evaporation apparatus (76), in addition to the dependences already recited, additionally depending on signals of the at least one ambient temperature sensor (17).

2. The ventilation apparatus (10) according to Claim 1,
**characterized in that** it comprises an input apparatus (24) for inputting a setpoint temperature of the respiratory gas flow (AF) in the proximal longitudinal end region (30a) of the respiratory gas conduit arrangement (30) and/or for inputting a setpoint moisture content of the respiratory gas and/or for inputting a setpoint respiratory gas flow.

3. The ventilation apparatus (10) according to Claim 2,
**characterized in that** it comprises an input apparatus (24) for inputting a setpoint temperature of the respiratory gas flow in the proximal longitudinal end region (30a) of the respiratory gas conduit arrangement (30), and comprises a data memory (19) which can be queried by the control apparatus (18) and in which a correlation between temperature values of the respiratory gas flow (AF) in the proximal longitudinal end region (30a) of the respiratory gas conduit arrangement (30) and associated setpoint moisture values of the respiratory gas is stored.

4. The ventilation apparatus (10) according to one of the preceding claims,
**characterized in that** at least one inspiratory conduit portion (36) of the respiratory gas conduit arrangement (30) comprises a conduit heating apparatus (37) with which the inspiratory conduit portion (36) is heatable; the control apparatus (18) preferably being embodied to control the operating power level of the conduit heating apparatus (37) depending on a predefined setpoint temperature of the respiratory gas flow (AF) in the proximal longitudinal end region (30a) of the respiratory gas conduit arrangement (30).

5. The ventilation apparatus (10) according to one of the preceding claims,
**characterized in that** the humidity sensor arrangement (66) is arranged downstream, in an inspiration direction of the respiratory gas flow (AF), from the respiratory gas source arrangement (15, 62).

6. The ventilation apparatus (10) according to one of the preceding claims,
**characterized in that** the control apparatus (18) is signal-transferringly connected to the evaporation apparatus (76) and/or to an energy supply thereof and/or to at least one energy sensor that is embodied to detect an energy delivered to the evaporation apparatus (76), in such a way that the energy and/or power level currently being delivered to the evaporation apparatus (76) is ascertainable by the control apparatus (18) from signals that are transferred via the signal-transferring connection.

7. The ventilation apparatus (10) according to one of the preceding claims,
**characterized in that** the respiratory gas conduit arrangement (30) comprises at its proximal end (30a) a coupling configuration (44a) for coupling the respiratory gas conduit arrangement (30) to a patient interface (31), for instance an endotracheal tube, a larynx mask, or a combination tube, and the like, which transfers respiratory gas between the respiratory gas conduit arrangement (30) and the patient (12); the control apparatus (18) being embodied to ascertain, depending on operating parameters of the ventilation apparatus (10), in particular of the respiratory gas flow (AF), a resultant state of the ventilation situation downstream, in an inspiration direction, from the coupling configuration (44a), and to modify the operating power level of the evaporation apparatus (76) depending on the ascertained result.

8. The ventilation apparatus (10) according to Claim 7,
**characterized in that** the resultant state of the ventilation situation encompasses a resultant temperature of the conduit wall of the patient interface (31) and/or of the respiratory gas flow (AF).

9. The ventilation apparatus (10) according to Claim 8,
**characterized in that** it comprises a data memory (19) which can be queried by the control apparatus (18) and in which operating parameter values of the respiratory gas flow (AF) are associated with resultant temperature values of the conduit wall of the patient interface (31) and/or of the respiratory gas flow (AF).

10. The ventilation apparatus (10) according to one of Claims 7 to 9,
**characterized in that** the control apparatus (18) ascertains the resultant state depending on: the temperature, detected by the proximal temperature sensor (48), of the respiratory gas flow (AF) in the proximal longitudinal end region (30a) of the respiratory gas conduit arrangement (30); the magnitude of the respiratory gas flow (AF) detected by the flow sensor (44); and the predefined setpoint moisture content of the respiratory gas.

11. The ventilation apparatus (10) according to one of Claims 7 to 10,
**characterized in that** the control apparatus (18) ascertains the resultant state depending on the ambient temperature detected by the ambient temperature sensor (17).

12. The ventilation apparatus (10) according to one of Claims 7 to 11,
**characterized in that** the control apparatus (18) ascertains the resultant state for an ascertainment location located closer to the proximal end (31a) of the patient interface (31) than to its distal end (31b); the control apparatus (18) preferably ascertaining the resultant state for the proximal end (31a) of the patient interface (31).

13. The ventilation apparatus (10) according to one of Claims 7 to 11,
**characterized in that** the control apparatus (18) ascertains the resultant state for a portion of the patient interface (31) which is located between the coupling configuration (44a) and the entry point (31c) of the patient interface (31) into the body of the patient (12).

14. The ventilation apparatus (10) according to one of Claims 7 to 13, incorporating Claim 4,
**characterized in that** the control apparatus is embodied to modify the temperature of the respiratory gas at the proximal temperature sensor (48), and for that purpose the operating power level of the conduit heating apparatus (37), depending on the ascertainment result.

## Revendications

1. Dispositif de ventilation (10) pour la ventilation artificielle d'un patient (12), comprenant :
- un ensemble de source de gaz respiratoire (15, 62) qui fournit un gaz respiratoire inspiratoire pour la respiration artificielle du patient (12),
- un dispositif de modification d'écoulement (16) qui est conçu pour générer et modifier en quantité un écoulement de gaz respiratoire inspiratoire (AF),
- un dispositif d'humidification (38) qui est conçu pour augmenter en quantité l'humidité absolue d'écoulement de gaz respiratoire inspiratoire (AF), dans lequel le dispositif d'humidification (38) présente à cet effet une réserve de liquide (40) et un dispositif d'évaporation à puissance variable (76),
- un agencement de conduite de gaz respiratoire (30) avec une extrémité longitudinale proximale (30a) plus proche du patient (12) en fonctionnement et avec une extrémité longitudinale distale (30b) plus éloignée du patient en fonctionnement, pour transporter le flux de gaz respiratoire inspiratoire (AF) du dispositif d'humidification (38) vers le patient (12),
- un capteur de température proximal (48) qui est conçu pour détecter la température du flux de gaz respiratoire (AF) dans la zone d'extrémité longitudinale proximale (30a) de l'agencement de conduite de gaz respiratoire (30),
- un agencement de capteur d'humidité (66) qui est conçu pour détecter au moins une valeur d'état d'humidité du flux de gaz respiratoire inspiratoire (AF), qui représente directement ou indirectement une humidité absolue du flux de gaz respiratoire inspiratoire (AF),
- un capteur de flux (44) qui est conçu pour détecter la valeur du flux de gaz respiratoire (AF),
- un dispositif de commande (18) qui est relié par transmission de signaux au capteur de température proximal (48), à l'agencement de capteur d'humidité (66) et au capteur de flux (44) et qui est conçu pour commander la puissance de fonctionnement du dispositif de vaporisation (76) en fonction d'une humidité de consigne prédéfinie du gaz respiratoire et en fonction de signaux du capteur de température proximal (48), de l'agencement de capteur d'humidité (66) et du capteur de flux (44),
dans lequel l'agencement de capteur d'humidité (66) est disposé en amont du dispositif d'humidification (38) dans le sens d'inspiration du flux de gaz respiratoire (AF) et est conçu pour détecter la valeur d'état d'humidité en amont du dispositif d'humidification (38), dans lequel le dispositif de ventilation (10) présente au moins un capteur de température ambiante (17) qui est conçu pour détecter la température ambiante (U) du dispositif de ventilation (10), dans lequel le dispositif de commande (18) est relié par transmission de signaux audit au moins un capteur de température ambiante (17) et est conçu pour commander la puissance de fonctionnement du dispositif de vaporisation (76), en plus des dépendances déjà mentionnées, également en fonction des signaux dudit au moins un capteur de température ambiante (17).

2. Dispositif de ventilation (10) selon la revendication 1,
**caractérisé en ce qu'**il comporte un dispositif d'entrée (24) pour entrer une température de consigne du flux de gaz respiratoire (AF) dans la zone d'extrémité longitudinale proximale (30a) de l'agencement de conduite de gaz respiratoire (30) ou/et pour entrer une humidité de consigne du gaz respiratoire ou/et pour entrer un flux de gaz respiratoire de consigne.

3. Dispositif de ventilation (10) selon la revendication 2,
**caractérisé en ce qu'**il présente un dispositif d'entrée (24) pour entrer une température de consigne du flux de gaz respiratoire dans la zone d'extrémité longitudinale proximale (30a) de l'agencement de conduite de gaz respiratoire (30) et présente une mémoire de données (19) pouvant être interrogée par le dispositif de commande (18), dans laquelle est enregistrée une relation entre des valeurs de température du flux de gaz respiratoire (AF) dans la zone d'extrémité longitudinale proximale (30a) de l'agencement de conduite de gaz respiratoire (30) et des valeurs d'humidité de consigne associées du gaz respiratoire.

4. Dispositif de ventilation (10) selon l'une des revendications précédentes,
**caractérisé en ce qu'**au moins une section de conduite inspiratoire (36) de l'agencement de conduite de gaz respiratoire (30) présente un dispositif de chauffage de conduite (37) avec lequel la section de conduite inspiratoire (36) peut être chauffée, dans lequel le dispositif de commande (18) est de préférence conçu pour commander la puissance de fonctionnement du dispositif de chauffage de conduite (37) en fonction d'une température de consigne prédéfinie du flux de gaz respiratoire (AF) dans la zone d'extrémité longitudinale proximale (30a) de l'agencement de conduite de gaz respiratoire (30).

5. Dispositif de ventilation (10) selon l'une des revendications précédentes,
**caractérisé en ce que** l'agencement de capteur d'humidité (66) est disposé en aval de l'ensemble de sources de gaz respiratoire (15, 62) dans le sens d'inspiration du flux de gaz respiratoire (AF).

6. Dispositif de ventilation (10) selon l'une des revendications précédentes,
**caractérisé en ce que** le dispositif de commande (18) est relié par transmission de signaux au dispositif de vaporisation (76) ou/et à une alimentation en énergie de celui-ci ou/et à au moins un capteur d'énergie qui est conçu pour détecter une énergie amenée au dispositif de vaporisation (76), de telle sorte que l'énergie ou/et la puissance actuellement amenée au dispositif de vaporisation (76) peut être déterminée par le dispositif de commande (18) à partir de signaux qui sont transmis par l'intermédiaire de la liaison par transmission de signaux.

7. Dispositif de ventilation (10) selon l'une des revendications précédentes,
**caractérisé en ce que** l'agencement de conduite de gaz respiratoire (30) comporte à son extrémité proximale (30a) une formation d'accouplement (44a) pour accoupler l'agencement de conduite de gaz respiratoire (30) à une interface patient (31) transmettant du gaz respiratoire entre l'agencement de conduite de gaz respiratoire (30) et le patient (12), telle qu'un tube endotrachéal, un masque laryngé ou un tube combiné et similaire, dans lequel le dispositif de commande (18) est conçu pour déterminer, en fonction de paramètres de fonctionnement du dispositif de ventilation (10), en particulier du flux de gaz respiratoire (AF), un état consécutif de la situation de ventilation dans la direction d'inspiration en aval de la formation d'accouplement (44a) et pour modifier la puissance de fonctionnement du dispositif de vaporisation (76) en fonction du résultat de la détermination.

8. Dispositif de ventilation (10) selon la revendication 7,
**caractérisé en ce que** l'état consécutif de la situation de ventilation comprend une température consécutive de la paroi de conduite de l'interface patient (31) ou/et du flux de gaz respiratoire (AF).

9. Dispositif de ventilation (10) selon la revendication 8,
**caractérisé en ce qu'**il présente une mémoire de données (19) pouvant être interrogée par le dispositif de commande (18), dans laquelle des valeurs des paramètres de fonctionnement du flux de gaz respiratoire (AF) sont associés à des valeurs de la température consécutive de la paroi de conduite de l'interface patient (31) ou/et du flux de gaz respiratoire (AF).

10. Dispositif de ventilation (10) selon l'une des revendications 7 à 9,
**caractérisé en ce que** le dispositif de commande (18) détermine l'état consécutif en fonction de la température du flux de gaz respiratoire (AF) détectée par le capteur de température proximal (48) dans la zone d'extrémité longitudinale proximale (30a) de l'agencement de conduite de gaz respiratoire (30), de la valeur du flux de gaz respiratoire (AF) détectée par le capteur de flux (44) et de l'humidité de consigne prédéterminée du gaz respiratoire.

11. Dispositif de ventilation (10) selon l'une des revendications 7 à 10,
**caractérisé en ce que** le dispositif de commande (18) détermine l'état consécutif en fonction de la température ambiante détectée par le capteur de température ambiante (17).

12. Dispositif de ventilation (10) selon l'une des revendications 7 à 11,
**caractérisé en ce que** le dispositif de commande (18) détermine l'état consécutif pour un lieu de détermination plus proche de l'extrémité proximale (31a) de l'interface patient (31) que de son extrémité distale (31b), dans lequel de préférence le dispositif de commande (18) détermine l'état consécutif pour l'extrémité proximale (31a) de l'interface patient (31).

13. Dispositif de ventilation (10) selon l'une des revendications 7 à 11,
**caractérisé en ce que** le dispositif de commande (18) détermine l'état consécutif pour une section de l'interface patient (31) qui est située entre la formation d'accouplement (44a) et le lieu d'entrée (31c) de l'interface patient (31) dans le corps du patient (12).

14. Dispositif de ventilation (10) selon l'une des revendications 7 à 13, en incluant la revendication 4,
**caractérisé en ce que** le dispositif de commande est conçu pour modifier, en fonction du résultat de la détermination, la température du gaz respiratoire au niveau du capteur de température proximal (48) et, à cet effet, la puissance de fonctionnement du dispositif de chauffage de conduite (37).
